# EUROPÄISCHE PATENTSCHRIFT

(11) **EP 3 235 878 B1**
(45) Veröffentlichungstag und Bekanntmachung des Hinweises auf die Patenterteilung: **20.03.2019**
(21) Anmeldenummer: 17165142.5
(22) Anmeldetag: 06.04.2017
(51) Int. Cl.: C09C 1/00, A61K 8/25, A61Q 1/02, A61Q 1/10, A61Q 1/12, A61Q 19/00, A61K 8/02, A61K 8/19, C09D 5/36, C09D 11/02, C08K 3/00, A61K 8/26, A61Q 1/06, A61Q 3/02, B01J 2/00, C09C 1/36, C09C 1/24, C09C 1/26, C09C 1/30, C09C 1/34

(54) **PIGMENTGEMISCH**
PIGMENT MIXTURE
MÉLANGE DE PIGMENTS

(30) Priorität: 11.04.2016 DE 102016004164
(43) Veröffentlichungstag der Anmeldung: 25.10.2017
(73) Patentinhaber: Merck Patent GmbH, 64293 Darmstadt (DE)
(72) Erfinder: Hamm, Lukas, 64853 OTZBERG (DE); Haferkorn, Nicole, 64319 PFUNGSTADT (DE); Jekel, Marita, 64287 DARMSTADT (DE)

(56) Entgegenhaltungen:
- EP-A1- 2 826 822
- EP-A2- 1 469 042
- WO-A1-00/15720
- WO-A2-2007/128576

## Beschreibung

Die vorliegende Erfindung betrifft ein Pigmentgemisch basierend auf mindestens drei Komponenten, sowie dessen Verwendung in Farben, Lacken, Druckfarben, Sicherheitsdruckfarben, Kunststoffen, keramischen Materialien, Gläsern, in kosmetischen Formulierungen, als Tracer, als Füllstoff und zur Herstellung von Pigmentpräparationen und Trockenpräparaten.

In kosmetischen Formulierungen werden in der Regel matte Absorptivpigmente eingesetzt um eine Körperfarbe der Formulierung zu erhalten. Diese müssen aufwendig vordispergiert werden, um sie in kosmetische Formulierungen einzuarbeiten. Werden matte Absorptivpigmente eingesetzt, wirken diese in kosmetischen Formulierungen oft stumpf und kreidig, so dass die Haut ein trockenes Erscheinungsbild bekommt.

Als eine spezielle Form von Pigmenten können Füllstoffe betrachtet werden. Bei Füllstoffen steht nicht die Funktion "Farbgebung" im Vordergrund. Bei technischen Füllstoffen sind vielmehr Faktoren wie die Verbesserung des Hautgefühls, die Erhöhung der mechanischen Stabilität, die Abriebfestigkeit, die Wetterstabilität oder auch die Herstellkosten ausschlaggebend für ihre Verwendung.

Die aus dem Stand der Technik bekannten Füllstoffe basierend auf SiO₂-Kugeln zeigen ein relativ gutes Hautgefühl, besitzen aber den Nachteil, dass sie ein zu hohes Streuvermögen aufweisen. Der Grund dafür ist in der Struktur der Metalloxidschichten der kugelförmigen Füllstoffe zu suchen. Die funktionellen Pigmente aus dem Stand der Technik bestehen in der Regel aus sehr kleinen Partikeln, d. h., sie weisen Partikelgrößen von 0,5 - 100 nm auf, die in gleichmäßiger Anordnung die Oberfläche der Trägerkugeln belegen. An der Schichtoberfläche wird das Licht reflektiert, wodurch Glanz hervorgerufen wird. Gleichzeitig findet aber auch ein erhebliches Maß von Streuung statt, da sich auf der Schicht Einzelpartikel bilden, die als starke Streuzentren wirken. Als Folge dieser zwei gegenläufigen Effekte (Glanz und Streuung) wirken die Pigmente weiß und unnatürlich auf der Haut.

Darüber hinaus sind die konventionellen Füllstoffe in der Regel farblos bzw. schwach gefärbt und wenig deckend. Gibt man also Füllstoffe zu einem System, welches auch Absorptivpigmente und/oder Effektpigmente enthält, wird durch die Füllstoffe sowohl der Effekt beeinflusst, als auch die Farbstärke sowie das Deckvermögen vermindert.

Breite Anwendung finden Füllstoffe auch in kosmetischen Formulierungen. Beispielsweise können Puder bis zu 50 % Füllstoffe basierend auf der Endformulierung enthalten. In Lippenstiften sind 10-15 % an Füllstoffen und in Emulsionen 2-6 % an Füllstoffen typische Werte. Kosmetische Füllstoffe erfüllen ganz unterschiedliche Funktionen: in Foundations vermeiden sie durch den sogenannten Mattierungseffekt einen unerwünschten Fettglanz auf der Haut, in Pudern helfen sie z.B. das Schüttverhalten oder die Auftragseigenschaften auf der Haut zu verbessern. In Deo-Produkten wird das hohe Flüssigkeitsaufnahmevermögen von einigen Füllstoffen genutzt.

Füllstoffe bzw. Pigmente in der Kosmetik müssen vor ihrer Anwendung im zu pigmentierenden System in eine Form gebracht werden, die eine leichte Dispergierung und eine reproduzierbare Farbe ermöglicht. Diese Vorbehandlungen der Pigmente, beispielsweise Mahlen, die entscheidend die Qualität des Endproduktes beeinflussen, sind zeitaufwendig und teuer. Nachteilig ist weiterhin, dass beim Benetzen die Farbe des Pigments verändert wird. Für kosmetische Formulierungen müssen die Pigmente zusätzlich ein gutes Hautgefühl besitzen, welches die klassischen Absorptivpigmente nur in geringem Umfang zeigen.

Füllstoffe auf der Basis sphärischer Teilchen, insbesondere SiO₂-Kugeln, werden zunehmend in der Kosmetik eingesetzt, da sie einerseits der menschlichen Haut ein natürliches Aussehen verleihen und andererseits Falten weniger sichtbar machen können.

Anorganische sphärische Füllstoffe, die mit einer farbgebenden Schicht belegt sind, sind beispielsweise aus den Offenlegungsschriften JP 62-288662, JP 11-139926, JP 11-335240 und DE 199 29 109 bekannt.

In der WO 00/15720 und EP 2826822 A1 werden Pigmentgemische basierend auf sphärischen SiO₂-Partikeln und/oder plättchenförmigen Substraten offenbart.

Aus der WO 99/66883 werden mit Metalloxiden, wie Titan-, Eisen- oder Zinkoxid, beschichtete SiO₂-Kugeln beschrieben, die eine finale SiO₂-Schicht aufweisen. Die so beschichteten SiO₂-Kugeln werden als Gemisch mit Interferenzpigmenten in kosmetischen Formulierungen eingesetzt. Absorptivpigmente, wie z.B. Eisenoxidpigmente, zeigen in kosmetischen Formulierungen häufig eine schlechte Dispergierbarkeit, ein trockenes und stumpfes Erscheinungsbild der Haut und/oder führen zu einem nicht befriedigendem Hautgefühl. Zur Behebung dieser Nachteile werden häufig verschiedene Füllstoffe eingesetzt oder die Eisenoxidpigmente werden einer Vorbehandlung unterworfen, damit sie sich in die kosmetischen Formulierungen dispergieren lassen, was mit Zeitaufwand und Kosten verbunden ist.

EP 2826822 A stellt eine Pigmentmischung bereit, die aus 2 Komponenten besteht und löst die Aufgabe, ein funktionelles Pigment bereit zu stellen, das neben einem guten Hautgefühl, eine reine, der Haut angepasste Farbe besitzt. EP 1 469 042 A offenbart ein Pigmentgemisch bestehend aus mindestens zwei Komponenten, wobei Komponente A Effektpigmente auf Basis von Glas-Plättchen und Komponente B organische und anorganische plättchenförmige, nadelförmige, sphärische oder kristalline Farbmittel und/oder Füllstoffe sind. WO 2007/128576 A offenbart ebenfalls ein Pigmentgemisch bestehend aus plättchenförmigen Pigmenten und sphärischen Füllstoffen, wobei die Mischung keine Hautfarbe zeigt.

Aufgabe der vorliegenden Erfindung ist es daher ein funktionelles und homogenes Pigmentgemisch bereit zu stellen, das neben einem guten Hautgefühl, gleichzeitig eine gute Dispergierbarkeit in kosmetischen Formulierungen, chemische und photochemische Stabilität und eine reine, der Haut angepasste Farbe sowie ein hohes Deckvermögen besitzt und nicht die oben genannten Nachteile aufweist.

Darüber hinaus soll das Pigmentgemisch im Hautauftrag als reines Pulver, in Cremes, Emulsionen, Foundations, u.ä., ein weiches und ebenmäßiges und natürliches Erscheinungsbild der Haut zeigen. Es ist weiterhin erwünscht, dass das Pigment insbesondere flüssigen und pastösen Zubereitungen eine leichte Erhöhung der Festigkeit verleiht und die Stabilität der Zubereitung garantiert. Dadurch wird die Verteilbarkeit der kosmetischen Zubereitungen auf der Haut deutlich erleichtert. So können hochviskose Cremes, also feste Foundations, hergestellt werden, die dennoch eine sehr gute Verteilbarkeit auf der Haut bzw. ein sehr gutes Neben diesen Produkteigenschaften ist eine einfache technische Herstellung des Pigmentgemisches eine weitere Aufgabe der Erfindung. Auch die Einstellung bestimmter Eigenschaften, wie z.B. das Deckvermögen und die gezielte einstellbare Farbintensität, des Pigmentgemisches sollen im Rahmen des Herstellungsverfahrens in einfacher Weise kontrolliert werden können.

Überraschenderweise wurde ein Pigmentgemisch in Form eines Dreikomponentensystems gefunden, dass die oben genannten Nachteile nicht aufweist, sondern sich durch ein sehr gutes Hautgefühl und ein hohes Deckvermögen auszeichnet und der Haut ein frisches und natürliches Erscheinungsbild verleiht, da matte Textur mit leichtem Glanz bzw. Leuchtkraft gezielt miteinander kombiniert werden.

Gegenstand der vorliegenden Erfindung ist ein Pigmentgemisch enthaltend die Komponenten A und B, wobei
- Komponente A ein Gemisch aus plättchenförmigen und sphärischen Substraten ist, das mit ein oder mehreren anorganischen Schichten, und/oder organischen Schichten belegt ist,
   und
- Komponente B kristalline oder amorphe Partikel ausgewählt aus der Gruppe der Metalloxide, Metallhydroxide, Metalloxyhalogenide, Berliner Blau oder deren Gemische sind.

Das erfindungsgemäße Dreikomponentengemisch zeichnet sich gegenüber den Pigmenten und Pigmentgemischen aus dem Stand der Technik durch
- ein mattes Erscheinungsbild mit definiertem Glanz und/oder definierter Farbintensität
- ein natürlicheres farblich angepasstes Erscheinungsbild der Haut
- eine leichtere Dispergierbarkeit
- eine verbesserte Verarbeitbarkeit
- ein in weiten Grenzen einstellbares Deckvermögen
- ein verbessertes Hautgefühl
- eine verbesserte Textur der kosmetischen Zubereitungen
- ein verbessertes Applikationsverhalten der kosmetischen Formulierungen
- einen erweiterten Farbraum, der sich bei matten Formulierungen darstellen lässt
- ein homogenes Pulver, da keine Separation in die Einzelkomponenten.
aus.

Ein weiterer Vorteil des erfindungsgemäßen Pigmentgemisches gegenüber den Produkten aus dem Stand der Technik ist die Homogenität des Pulvers. Während beim separaten Einsatz von Füllstoffen, Absorptivpigmenten und Effektpigmenten oft eine Separation stattfindet, ist durch den erfindungsgemäßen Prozess ein homogenes Produkt gewährleistet, d.h., alle Komponenten des erfindungsgemäßen Pigmentgemisches sind untrennbar miteinander verbunden und eine Separation in die Einzelkomponenten ist nicht möglich.

Weiterhin zeigt das erfindungsgemäße Pigmentgemisch den gewünschten Einfluss auf die Textur und Stabilität, d. h. es wirkt leicht erhöhend auf die Viskosität von Emulsionen bzw. die Festigkeit von Foundations, ohne die Applikationseigenschaften ungünstig zu beeinflussen und es behält gleichzeitig die Stabilität der Zubereitung bei.

Gegenstand der Erfindung ist weiterhin die Verwendung des erfindungsgemäßen Pigmentgemisches in Farben, Lacken, vorzugsweise in Industrielacken, Druckfarben, Sicherheitsdruckfarben, Kunststoffen, keramischen Materialien, Gläsern, als Tracer, als Füllstoff und insbesondere in kosmetischen Formulierungen. Weiterhin sind die erfindungsgemäßen Pigmente auch zur Herstellung von Pigmentpräparationen sowie zur Herstellung von Trockenpräparaten, wie z. B. Granulaten, Pearlets, Chips, Pellets, Würstchen, Briketts, etc., geeignet. Die Trockenpräparate finden insbesondere Anwendung in Druckfarben und in der Kosmetik.

Bei den plättchenförmigen Substraten der Komponente A handelt es sich vorzugsweise um transparente plättchenförmige Substrate. Bevorzugte Substrate sind Schichtsilikate. Insbesondere geeignet sind natürlicher oder synthetischer Glimmer, Talkum, Kaolin, plättchenförmige Fe₂O₃-, Fe₃O₄-, Al₂O₃-, BiOCl-, Glas-, SiO₂-, TiO₂-, BN-, Oxinitrid-, Nitrid-, Graphitplättchen, Fischsilber, synthetische trägerfreie Plättchen oder andere vergleichbare Materialien.

Es können auch Gemische unterschiedlicher Plättchen eingesetzt werden. Besonders bevorzugte Plättchengemische der Komponente A bestehen aus
Glimmerplättchen + SiO₂-Plättchen
Glimmerplättchen + Al₂O₃-Plättchen
Glimmerplättchen + Glas-Plättchen
Glimmerplättchen + TiO₂-Plättchen
Glimmerplättchen + Oxinitrid-Plättchen
Glimmerplättchen + Nitrid-Plättchen
Glimmerplättchen + Fischsilber
Glimmerplättchen + Graphitplättchen
Glimmerplättchen + BiOCI
SiO₂-Plättchen + Al₂O₃-Plättchen
Glasplättchen + SiO₂-Plättchen.

Die Größe der plättchenförmigen Substrate ist an sich nicht kritisch und kann auf den jeweiligen Anwendungszweck abgestimmt werden. In der Regel haben die plättchenförmigen Substrate eine Dicke zwischen 0,05 und 1,5 µm, insbesondere zwischen 0,1 und 1 µm. Die Ausdehnung in den beiden anderen Bereichen beträgt üblicherweise zwischen 1 und 250 µm, vorzugsweise zwischen 2 und 200 µm, und insbesondere zwischen 5 und 60 µm. Es können auch Substrate unterschiedlicher Partikelgrößen eingesetzt werden. Besonders bevorzugt ist ein Gemisch aus Glimmerfraktionen von N-Glimmer (10-60 µm), F-Glimmer (5-20 µm) und M-Glimmer (<15 µm). Weiterhin bevorzugt sind N- und S-Fraktionen (10-130 µm) und F- und S-Fraktionen (5-130 µm).

Die plättchenförmigen Substrate der Komponente A besitzen in einer ganz besonders bevorzugten Ausführungsform eine Teilchengröße von 0,1 - 100 µm, insbesondere von 0,3 - 60 µm und ganz besonders bevorzugt von 0,5 - 15 µm.

Geeignete sphärische Basissubstrate für Komponente A sind sphärische Partikel, wie sie z. B. im Handel angeboten werden, u.a. von Sunjin Chemicals, Kobo, Ikeda, Asahi Glass, Miyoshi, Sinoenergy Group, Omega Materials, 3M, ABC NanoTech, China New Technology, PQ Corporation, Sibelco oder Evonik. Bevorzugte sphärische Partikel sind ausgewählt aus der Gruppe Magnesiumsilikat, Aluminiumsilikat, Alkalialuminiumsilikate, Erdalkalialuminiumsilikate und deren Kombinationen, SiO₂-Kugeln, Glaskugeln, Glashohlkugeln, Aluminiumoxid, keramischen Kugeln und polymere Kugeln aus Ethylen/ Acrylsäure-Copolymeren, Ethylen/ Methacrylat-Copolymeren, HDI/Trimethylol Hexyllactone-Coplymeren, Nylon, Polyacrylate, Polymethylmethacrylat-Coplymere, Polyethylen, Polymethylsilsesquioxane und deren Kombinationen.

Besonders bevorzugte sphärische Substrate sind SiO₂-Kugeln, wie sie beispielsweise im Handel unter den Markennamen
SUNSIL, MSS-500, SHERON, SUNSPHERE, Silicabeads SB, OMEGA-SIL, OMEGA-Spheres, SPHERICEL, Q-Cel, Ceramic Microspheres, Glasbubbles iM-K, SILNOS, SS-T4, SS-S3, SORBOSIL, AEROSIL, Flo-Beads, BPD, Daiamid, MSP, TOSPEARL, Ronaspheres vertrieben werden.

Die per Stickstoffabsorption bestimmte BET-Oberfläche der geeigneten sphärischen Basispartikel beträgt in der Regel 1 - 1000, vorzugsweise 10 - 750, insbesondere 20 - 550 m²/g. Die BET-Oberfläche in dieser Patentanmeldung wird bestimmt nach DIN ISO 9277: 2003-05.

Die sphärischen Basissubstrate der Komponente A besitzen vorzugsweise einen Partikeldurchmesser im Bereich von 0,1 - 100 µm, insbesondere von 0,3 - 60 µm und ganz besonders bevorzugt von 0,5 - 15 µm.

Die sphärischen und die plättchenförmigen Substrate der Komponente A können in jedem Mengenverhältnis miteinander gemischt werden. Vorzugsweise beträgt das Verhältnis Kugeln : Plättchen = 99 : 1 bis 1 : 99, insbesondere 90 : 10 bis 10 : 90, ganz besonders bevorzugt 85 : 15 bis 25 : 75. Durch einen erhöhten Anteil an Plättchen kann der Glanz und die Farbintensität erhöht werden, während ein größerer Anteil an Kugeln sich vorteilhaft auf das Hautgefühl und die Homogenität im Auftrag auswirkt.

Besonders bevorzugte Substratgemische der Komponente A werden nachfolgend genannt:
- Natürliche Glimmerplättchen + SiO₂-Kugeln
- Synthetische Glimmerplättchen + SiO₂-Kugeln
- Al₂O₃-Plättchen + SiO₂-Kugeln
- SiO₂-Plättchen + SiO₂-Kugeln
- Glasplättchen + SiO₂-Kugeln
- Natürliche Glimmerplättchen + Glas-Kugeln
- Synthetische Glimmerplättchen + Glas-Kugeln
- Al₂O₃-Plättchen + Glas-Kugeln
- SiO₂-Plättchen + Glas-Kugeln
- Glasplättchen + Glas-Kugeln.

Die Komponente A bestehend aus sphärischen und plättchenförmigen Partikeln wird nachfolgend mit ein oder mehreren, vorzugsweise ein oder zwei Schichten, vorzugsweise anorganischen Schichten belegt. Bei der anorganischen Schicht handelt es sich vorzugsweise um absorbierende und nicht absorbierende Oxide oder Berliner Blau.

Die anorganische Schicht besteht vorzugsweise aus Oxiden ausgewählt aus der Gruppe Fe₂O₃, Fe₃O₄, FeOOH, Cr₂O₃, ZrO₂, Al₂O₃, SnO₂, SiO₂, ZnO, TiO₂, Titansuboxiden. Das TiO₂ kann dabei in der Rutil- oder in der Anatasmodifikation vorliegen.

Bei den absorbierenden Schichten handelt es sich vorzugsweise um Schichten aus Fe₂O₃, Fe₃O₄, Cr₂O₃, FeOOH, TiO₂/Fe₂O₃-Mischschicht, Pseudobrookit, Ilmenit, Berliner Blau oder Karminrot.

In einer bevorzugten Ausführungsform weist die Komponente A mindestens eine absorbierende und eine nicht absorbierende Schicht auf der Oberfläche des Plättchen/Kugel-Gemisches auf.

Die Dicke der einzelnen Schichten auf dem Substratgemisch der Komponente A ist wesentlich für die optischen Eigenschaften des finalen Produkts. Insbesondere die 1. Schicht auf der Oberfläche des Kugel-/Plättchengemisches beeinflusst wesentlich die Farbeigenschaften.

Die Dicke der ersten Schicht beträgt vorzugsweise 10 - 1000 nm, insbesondere 30 - 600 nm und besonders bevorzugt 50 - 300 nm. Die Schichtdicken der 1., der 2. und der weiteren Schichten, sofern vorhanden, können gleich oder verschieden sein. Vorzugsweise besitzen sie ähnliche bzw. gleiche Schichtdicken und können auch in der Abfolge ausgetauscht werden.

Mit Hilfe der Beschichtung (ein oder mehrere Schichten) ist es möglich Farbe, Glanz, Deckvermögen in weiten Grenzen zu variieren bzw. einzustellen.

Besonders bevorzugte Gemische (Kugeln + Plättchen) der Komponente A weisen folgende Schichtenfolgen auf der Oberfläche auf:
Substratgemisch + Fe₂O₃ (1. Schicht) + TiO₂ (2. Schicht)
Substratgemisch + Fe₂O₃ (1. Schicht) + SnO₂ (2. Schicht)
Substratgemisch + Fe₂O₃ (1. Schicht) + SiO₂ (2. Schicht)
Substratgemisch + Fe₃O₄ (1. Schicht) + TiO₂ (2. Schicht)
Substratgemisch + Fe₃O₄ (1. Schicht) + SnO₂ (2. Schicht)
Substratgemisch + Fe₃O₄ (1. Schicht) + SiO₂ (2. Schicht)
Substratgemisch + Cr₂O₃ (1. Schicht) + TiO₂ (2. Schicht)
Substratgemisch + Cr₂O₃ (1. Schicht) + SnO₂ (2. Schicht)
Substratgemisch + Cr₂O₃ (1. Schicht) + SiO₂ (2. Schicht)
Substratgemisch + TiO₂ (1. Schicht) + Berliner Blau (2. Schicht)
Substratgemisch + TiO₂ (1. Schicht) + Karminrot (2. Schicht)
Substratgemisch + SnO₂ (1. Schicht) + TiO₂ (2. Schicht)
Substratgemisch + TiO₂ (1. Schicht)
Substratgemisch + Fe₂O₃ (1. Schicht)
Substratgemisch + FeOOH (1. Schicht)
Substratgemisch + TiO₂ (1. Schicht) + Fe₂O₃ (2. Schicht)
Substratgemisch + Berliner Blau (1. Schicht)
Substratgemisch + Cr₂O₃ (1. Schicht)
Substratgemisch + Fe₂O₃ (1. Schicht) + TiO₂ (2. Schicht) + SiO₂ (3. Schicht)
Substratgemisch + TiO₂ (1. Schicht) + SiO₂ (2. Schicht) + TiO₂ (3. Schicht)

Bei der Komponente B handelt es sich vorzugsweise um kristalline oder amorphe Partikel aus der Gruppe Karminrot, Berliner Blau, Metalloxid, wie z.B. TiO₂, Fe₂O₃, Cr₂O₃, Fe₃O₄, Titansuboxide, wie z.B. TiOₓ wobei x = 1,5-1,95 ist, Metallhydoxid, wie z.B. FeOOH, Metalloxyhalogenid, wie z.B. BiOCI bzw. deren Gemische.

Die Komponente B hat vorzugsweise Partikelgrößen im Bereich von 0,1 - 100 µm, insbesondere 0,3 - 30 µm und ganz besonders bevorzugt von 0,5 - 10 µm.

Besonders bevorzugt sind Pigmentgemische, in denen alle Basissubstrate, d.h. Plättchen, Kugeln der Komponente A und die kristallinen oder amorphen Partikel der Komponente B ähnliche Partikelgrößen aufweisen. Insbesondere bevorzugt sind Pigmentgemische, wo die drei Basissubstrate der Komponenten A und B alle eine Partikelgröße bzw. einen Partikeldurchmesser im Bereich von 0,1 - 100 µm, insbesondere 0,3 - 30 µm und ganz besonders bevorzugt von 0,5 - 15 µm aufweisen.

Das erfindungsgemäße Pigmentgemisch lässt sich leicht herstellen durch die Belegung des Substratgemisches der Komponente A mit ein oder mehreren Schichten, z.B. Metalloxidschicht(en), Berliner Blau und Mischen der Komponente A mit Komponente B. Die Belegung des Substratgemisches der Komponente A erfolgt vorzugsweise nasschemisch. Zu der wässrigen Suspension der Komponente A wird dann die wässrige Suspension der Komponente B zu gegeben. Es ist aber auch möglich, dass nach der Belegung des Substratgemisches der Komponente A das Produkt abgetrennt und aufgearbeitet wird und anschließend das getrocknete Produkt der Komponente A mit der Suspension der Komponente B gemischt wird. Eine weitere Möglichkeit besteht darin, die Suspension einer Komponente B herzustellen, anschließend das Substratgemisch der Komponente A zuzugeben und dieses Gemisch danach in einer Eintopfsynthese zu belegen.

Die Metalloxidschichten auf die Oberfläche des Substratgemisches der Komponente A werden vorzugsweise nasschemisch aufgebracht, wobei die zur Herstellung von Perlglanzpigmenten entwickelten nasschemischen Beschichtungsverfahren angewendet werden können. Derartige Verfahren sind z. B. beschrieben in U.S. 3087828, U.S. 3087829, U.S. 3553001, DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017, DE 196 18 568, EP 0 659 843, oder auch in weiteren dem Fachmann bekannten Patentdokumenten und sonstigen Publikationen

Bei der Nassbeschichtung werden die sphärischen und plättchenförmigen Basissubstrate der Komponente A in Wasser suspendiert und mit einem oder mehreren hydrolysierbaren Metallsalzen bei einem für die Hydrolyse geeigneten pH-Wert versetzt, der so gewählt wird, dass die Metalloxide bzw. Metalloxidhydrate direkt auf den Plättchen und Kugeln ausgefällt werden, ohne dass es zu Nebenfällungen kommt. Der pH-Wert wird üblicherweise durch gleichzeitiges Zudosieren einer Base oder Säure konstant gehalten. Anschließend wird das so beschichtete Substratgemisch (=Komponente A) mit Komponente B, vorzugsweise als Suspension in Wasser, versetzt, und gemischt und das finale Produkt abgetrennt, gewaschen und getrocknet und ggf. geglüht, wobei die Glühtemperatur in Hinblick auf die jeweils vorliegende Beschichtung optimiert werden kann. In der Regel liegen die Glühtemperaturen zwischen 250 und 900 °C, vorzugsweise zwischen 450 und 700 °C. Falls gewünscht kann das Substratgemisch der Komponente A nach Aufbringen einzelner Beschichtungen abgetrennt, getrocknet oder geglüht werden, um dann zur Auffällung der weiteren Schichten wieder resuspendiert zu werden.

Werden Titandioxid und Eisenoxid in dem erfindungsgemäßen Pigmentgemisch eingesetzt, lassen sich abhängig von der Glühtemperatur Farbtöne in Gelb, Braun und Rot erzielen. Insbesondere bei Glühtemperaturen > 700 °C sind die Gelbtöne vorherrschend, da sich bei Komponente A Mischoxide aus Titanoxid und Eisenoxid, z.B. Pseudobrookit, bilden können.

Die Herstellung der Fe₃O₄-Schicht kann z. B. erfolgen durch Reduktion der Fe₂O₃-Schicht mit Ammoniak, Wasserstoff sowie auch Kohlenwasserstoffen und Kohlenwasserstoff/Ammoniak-Gemischen wie z.B. beschrieben in EP-A-0 332 071, DE 19 51 696.8 und DE 19 51 697.7. Vorzugsweise findet die Reduktion in einer Formiergasatmosphäre (N₂/H₂), insbesondere bei 92% N₂/8% H₂ oder 96% N₂/4% H₂, statt. Die Reduktionstemperatur beträgt vorzugsweise 400 bis 700 °C, insbesondere 500 bis 600 °C.

Für das Aufbringen einer finalen SiO₂-Schicht wird bevorzugt das in der DE 196 18 569 beschriebene Verfahren verwendet. Zur Herstellung der SiO₂-Schicht wird vorzugsweise Natrium- oder Kaliumwasserglaslösung eingesetzt.

Weiterhin kann die Beschichtung auch in einem Wirbelbettreaktor durch Gasphasenbeschichtung erfolgen, wobei z.B. die in EP 0 045 851 und EP 0 106 235 zur Herstellung von Perlglanzpigmenten vorgeschlagenen Verfahren entsprechend angewendet werden können.

Der Farbton des finalen Pigmentgemisches kann in weiten Grenzen durch unterschiedliche Wahl der Belegungsmengen bzw. der daraus resultierenden Schichtdicken variiert werden. Die Feinabstimmung für einen bestimmten Farbton kann über die reine Mengenwahl hinaus durch visuell oder messtechnisch kontrolliertes Anfahren der gewünschten Farbe erreicht werden.

Durch die gemeinsame Aufarbeitung der Suspension enthaltend Komponente A und Komponente B lassen sich die oben angegebenen Eigenschaften einstellen und gleichzeitig ist die Homogenität des Pigmentgemisches gewährleistet und eine Entmischung wird durch die Partikelform und die strukturierte Oberfläche der beschichteten Substrate des Pigmentgemisches vermieden.

Gegenstand der Erfindung ist sind auch die Verfahren zur Herstellung es erfindungsgemäßen Pigmentgemisches.

Gegenstand der Erfindung sind weiterhin Pigmentgemische hergestellt nach den erfindungsgemäßen Verfahren, die sich dadurch auszeichnen, dass bei der nasschemischen Beschichtung zu der wässrigen Suspension der Komponente A die wässrige Suspension der Komponente B gegeben wird und die Suspension enthaltend Komponenten A und B aufgearbeitet wird, oder dass nach der nasschemischen Belegung des Substratgemisches der Komponente A das Produkt abgetrennt und aufgearbeitet wird und anschließend das getrocknete Produkt der Komponente A mit der Suspension der Komponente B gemischt wird und die Suspension dann aufgearbeitet wird, oder dass zu der Suspension der Komponente B das Substratgemisch der Komponente A gegeben wird und das Gemisch aus Komponente B und Substratgemisch der Komponente A gemeinsam nasschemisch mit ein oder mehreren organischen oder anorganischen Schichten belegt wird, und dann aufgearbeitet wird.

Unter Beschichtung(en) sind in dieser Patentanmeldung die vollständige Belegung der jeweiligen Oberfläche der Basissubstrate zu verstehen.

Die Komponente A und die Komponente B können in jedem Verhältnis miteinander gemischt werden. Bevorzugt für die Farboptimierung in der jeweiligen Anwendung ist ein Mischungsverhältnis (Gewichtsteile) von Komponente A zu Komponente B von 99 : 1 bis 1 : 99, insbesondere von 90 : 10 bis 10 : 90 und ganz besonders bevorzugt von 80 : 20 bis 20 : 80 Das Mischungsverhältnis bezieht sich hierbei auf die Masse.

Das erfindungsgemäße Pigmentgemisch weist in der Regel eine Ölzahl von 10 - 200 g / 100g, insbesondere von 20 - 200 g / 100 g, ganz besonders bevorzugt von 50 - 150 g / 100 g, auf. Die Ölzahl in dieser Patentanmeldung wird bestimmt nach DIN ISO 787/5-1980 (E).

Die erfindungsgemäßen Pigmentgemische verbessern insbesondere die Textur von Kosmetika in der Weise, dass sie ein erleichtertes Auftragen und eine gleichmäßigere Verteilung auf der Haut erzielen und das Hautgefühl verbessern. Da das erfindungsgemäße Pigmentgemisch auf nichttoxischer mineralischer Basis aufgebaut ist und überwiegend anorganische Komponenten enthält, ist es sehr gut auf der Haut verträglich.

Zur Erhöhung der Licht-, Wasser- und Wetterstabilität empfiehlt es sich häufig, in Abhängigkeit vom Einsatzgebiet, das Pigmentgemisch einer Nachbeschichtung oder Nachbehandlung zu unterziehen. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage. Durch diese Nachbeschichtung wird die chemische und photochemische Stabilität weiter erhöht oder die Handhabung des Pigmentgemisches, insbesondere die Einarbeitung in unterschiedliche Medien erleichtert. Zur Verbesserung der Benetzbarkeit, Dispergierbarkeit und/oder Verträglichkeit mit den Anwendermedien können funktionelle Beschichtungen aus Al₂O₃ oder ZrO₂ oder deren Gemische auf die Pigmentoberfläche aufgebracht werden. Weiterhin sind organische Nachbeschichtungen möglich, z.B. mit Silanen, wie beispielsweise beschrieben in der EP 0090259, EP 0 634 459, WO 99/57204, WO 96/32446, WO 99/57204, U.S. 5,759,255, U.S. 5,571,851, WO 01/92425 oder in J.J. Ponjee, Philips Technical Review, Vol. 44, No. 3, 81 ff. und P.H. Harding J.C. Berg, J. Adhesion Sci. Technol. Vol. 11 No. 4, S. 471-493.

Gegenstand der Erfindung ist auch ein Verfahren zur Herstellung des erfindungsgemäßen Pigmentgemisches, indem man plättchenförmige Substrate mit sphärischen Partikeln mischt und vorzugsweise nasschemisch mit ein oder mehreren absorbierenden oder nicht absorbierenden Schichten, z.B. Metalloxide, belegt und das beschichtete Substrat/Kugel-Gemisch (Komponente A) mit kristallinen oder amorphen Partikeln (Komponente B) mischt, und das Gemisch aus den Komponenten A und B gemeinsam aufarbeitet, und das finale Gemisch trocknet und optional bei Temperaturen von 150 - 1000 °C glüht.

Das erfindungsgemäße Pigmentgemisch ist mit einer Vielzahl von Farbsystemen kompatibel, vorzugsweise aus dem Bereich der Lacke, Farben und Druckfarben. Für die Herstellung der Druckfarben für z. B. den Tiefdruck, Flexodruck, Offsetdruck, Offsetüberdrucklackierung, ist eine Vielzahl von Bindern, insbesondere wasserlösliche Typen, geeignet, wie sie z. B. von den Firmen BASF, Marabu, Pröll, Sericol, Hartmann, Gebr. Schmidt, Sicpa, Aarberg, Siegwerk, GSB-Wahl, Follmann, Ruco oder Coates Screen INKS GmbH vertrieben werden. Die Druckfarben können auf Wasserbasis oder Lösemittelbasis aufgebaut sein.

Mit dem erfindungsgemäßen Pigmentgemisch lassen sich besonders wirksame Effekte wie Glättung von Oberflächen und Ausgleichung von Unebenheiten (Falten, Poren, Vertiefungen, Mikrorisse, etc.) in den verschiedenen Anwendungsmedien erzielen, z. B. in kosmetischen Formulierungen, wie z. B. Nagellacken, Lippenstiften, Presspudern, Gelen, Lotionen, Seifen, Zahnpasta, in Lacken, in Industrielacken und Pulverlacken, sowie in Kunststoffen und in der Keramik.

Aufgrund des guten Hautgefühls und der sehr guten Hautadhäsion ist das erfindungsgemäße Pigmentgemisch insbesondere als Füllstoff in der dekorativen Kosmetik, aber auch für Personal Care Anwendungen, wie z.B. Body Lotions, Emulsionen, Seifen, Shampoos, BB Cremes, CC Cremes, DD Cremes, etc., geeignet. Das erfindungsgemäße Pigmentgemisch weist eine stabilisierende Wirkung auf, wie sie z.B. in Cremes, Emulsionen und Lotions, gewünscht wird.

Beim Einsatz in Kunstoffen z.B. in Spritzgussteilen wirkt sich der Einsatz positiv auf die Vermeidung von Fließlinien oder Sink-Marks aus.

Es versteht sich von selbst, dass für die verschiedenen Anwendungszwecke das erfindungsgemäße Pigmentgemisch auch vorteilhaft in Abmischung mit z. B.
- Metalleffektpigmenten, z. B. auf der Basis von Eisen- oder Aluminiumplättchen;
- Perlglanzpigmenten auf der Basis von metalloxidbeschichteten synthetischen Glimmer-Plättchen, natürlichen Glimmerplättchen, Glas-Plättchen, Al₂O₃-Plättchen, Fe₂O₃-Plättchen oder SiO₂-Plättchen;
- Interferenzpigmenten auf der Basis von metalloxidbeschichteten synthetischen Glimmer-Plättchen, natürlichen Glimmerplättchen, Glas-Plättchen, Al₂O₃-Plättchen, Fe₂O₃-Plättchen oder SiO₂-Plättchen;
- goniochromatischen Pigmenten;
- Mehrschichtpigmenten (enthaltend vorzugsweise 2, 3, 4, 5 oder 7 Schichten) auf der Basis von metalloxidbeschichteten synthetischen Glimmer-Plättchen, natürlichen Glimmerplättchen, Glas-Plättchen, Al₂O₃-Plättchen, Fe₂O₃-Plättchen oder SiO₂-Plättchen;
- organischen Farbstoffen;
- organischen Pigmenten;
- anorganischen Pigmenten, wie z.B. transparenten und deckenden Weiß-, Bunt- und Schwarzpigmenten;
- plättchenförmigen Eisenoxiden;
- Ruß
eingesetzt werden kann.

Das erfindungsgemäße Pigmentgemisch kann in jedem Verhältnis mit handelsüblichen Pigmenten und/oder weiteren handelsüblichen Füllstoffen gemischt werden.

Als handelsübliche Füllstoffe sind z.B. zu nennen natürlicher und synthetischer Glimmer, Nylon Powder, reine oder gefüllte Melaminharze, Talkum, Gläser, Kaolin, Oxide oder Hydroxide von Aluminium, Magnesium, Calcium, Zink, BiOCI, Bariumsulfat, Calciumsulfat, Calciumcarbonat, Magnesiumcarbonat, Kohlenstoff, Bornitrid sowie physikalische oder chemische Kombinationen dieser Stoffe. Bezüglich der Partikelform des Füllstoffes gibt es keine Einschränkungen. Sie kann den Anforderungen gemäß z.B. plättchenförmig, sphärisch oder nadelförmig sein.

Selbstverständlich kann das erfindungsgemäße Pigmentgemisch in den Formulierungen auch mit jeder Art von kosmetischen Roh- und Hilfsstoffen kombiniert werden. Dazu gehören u.a. Öle, Fette, Wachse, Filmbildner, Konservierungsmittel und allgemein anwendungstechnische Eigenschaften bestimmende Hilfsstoffe, wie z.B. Verdicker und rheologische Zusatzstoffe, wie z.B. Bentonite, Hektorite, Siliziumdioxide, Ca-Silicate, Gelatinen, hochmolekulare Kohlenhydrate und/oder oberflächenaktive Hilfsmittel, etc.

Die das erfindungsgemäße Pigmentgemisch enthaltende Formulierung kann dem lipophilen, hydrophilen oder hydrophoben Typ angehören. Bei heterogenen Formulierungen mit diskreten wässrigen und nichtwässrigen Phasen kann das erfindungsgemäße Pigmentgemisch in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 4 und 10 liegen.

Den Konzentrationen des erfindungsgemäßen Pigmentgemisches in der Formulierung sind keine Grenzen gesetzt. Sie können - je nach Anwendungsfall - zwischen 0,001 (rinse-off-Produkte, z.B. Duschgele) - und 60 % liegen. Das erfindungsgemäße Pigmentgemisch kann weiterhin auch mit kosmetischen Wirkstoffen kombiniert werden. Geeignete Wirkstoffe sind z.B. Insect Repellents, anorganische UV-Filter, wie z.B. TiO₂, UV A/BC-Schutzfilter (z.B. OMC, B3, MBC), auch in verkapselter Form, Anti-Ageing-Wirkstoffe, Vitamine und deren Derivate (z.B. Vitamin A, C, E, etc.), Selbstbräuner (z.B. DHA, Erytrolose, u.a.) sowie weitere kosmetische Wirkstoffe, wie z.B. Bisabolol, LPO, Ectoin, Emblica, Allantoin, Bioflavanoide und deren Derivate.

Organische UV-Filter werden in der Regel in einer Menge von 0,5 - 10 Gew.%, vorzugsweise 1-8 Gew.%, anorganische UV-Filter von 0,1 - 30 Gew.% eingesetzt, bezogen auf die kosmetische Formulierung.

Die Formulierungen können darüber hinaus weitere übliche hautschonende oder hautpflegende Wirkstoffe enthalten, wie z.B. Aloe Vera, Avocadoöl, Coenzym Q10, Grüner Tee Extrakt und auch Wirkstoffkomplexe.

Gegenstand der vorliegenden Erfindung sind ebenfalls Formulierungen, insbesondere kosmetische Formulierungen, die neben dem erfindungsgemäßen Pigmentgemisch mindestens einen Bestandteil ausgewählt aus der Gruppe der Absorptionsmittel, Adstringenzien, antimikrobiellen Stoffe, Antioxidantien, Antiperspirantien, Antischaummitteln, Antischuppenwirkstoffe, Antistatika, Bindemittel, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Füllstoffen, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Aloe Vera, Avocadoöl, Coenzym Q10, Grüner Tee Extrakt, Viskositätsreglern, Parfüm und Vitaminen enthalten.

Gegenstand der Erfindung ist auch die Verwendung des erfindungsgemäßen Pigmentgemisches in Farben, Lacken, Druckfarben, Sicherheitsdruckfarben, Kunststoffen, keramischen Materialien, Glasuren, Gläsern, als Tracer, in kosmetischen Formulierungen und zur Herstellung von Pigmentpräparationen und Trockenpräparaten.

Die nachfolgenden Beispiele sollen die Erfindung näher erläutern ohne sie jedoch zu beschränken.

### Beispiele

### Beispiel 1: Pigmentgemisch mit blauer Körperfarbe

### Ansatz 1A:

Es werden 8 g natürliche Glimmerplättchen der Fa. Merck KGaA (Teilchengröße <15 µm) zusammen mit 80 g Siliziumdioxid-Kugeln der Firma ABC Nanotech (Durchmesser: 2-4 µm) in 800 ml VE-Wasser gerührt.

Diese Suspension wird unter Rühren auf die Reaktionstemperatur von 75 °C erhitzt. Der pH-Wert wird mittels 10 %iger Schwefelsäure auf 2,1 eingestellt dann 2200 g einer 25 %igen Titantetrachlorid-Lösung zudosiert. Der pH-Wert wird mit 20 %iger Natronlauge konstant gehalten. Nach vollständiger Zugabe wird 5 Minuten nachgerührt und dann der pH-Wert mit 10 %iger Schwefelsäure auf 4,3 eingestellt. Anschließend werden gleichzeitig zwei Lösungen 1 und 2 zugeben, während der pH-Wert mit einer 10 %igen Ammoniumcarbonat-Lösung konstant gehalten wird. Lösung 1 wird aus 60 g Kaliumhexacyanoferrat(III) und 1300 g VE-Wasser hergestellt. Lösung 2 besteht aus 76 g Eisensulfat Heptahydrat und 1250 g VE-Wasser. Nach vollständiger Zugabe werden 15 min nachgerüht und dann der pH-Wert mit 20 %iger Natronlauge auf pH 6 gestellt.

### Ansatz 1B:

Es werden 900 ml VE-Wasser unter Rühren auf die Reaktionstemperatur von 73 °C erhitzt und dann der pH-Wert mit 10 %iger Schwefelsäure 4,3 eingestellt. Anschließend werden gleichzeitig zwei Lösungen 1 und 2 zugegeben, während der pH-Wert mit einer 10 %igen Ammoniumcarbonat-Lösung konstant gehalten wird. Lösung 1 wird aus 165 g Kaliumhexacyanoferrat(III) und 2100 g VE-Wasser hergestellt. Lösung 2 besteht aus 209 g Eisensulfat Heptahydrat und 1950 g VE-Wasser. Danach wird der pH-Wert mit 20 %iger Natronlauge auf pH 6 gestellt.

Nachdem beide Ansätze beendet sind, werden die Suspensionen der Ansätze 1A und 1B gemischt, abfiltriert, gewaschen und bei 150 °C getrocknet und dann durch ein Sieb der Maschenweite 24 µm gesiebt. Man erhält ein blaues körperfarbenes Pigmentgemisch mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 1A/1B lassen sich Deckvermögen, Hautgefühl und Chroma beliebig einstellen.

### Beispiel 1a:

Ist das Verhältnis von 1A : 1B = 8 : 2, erhält man besonders hochchromatische Pigmentgemische mit außerordentlich gutem Hautgefühl und moderatem Deckvermögen.

### Beispiel 1b:

Ist das Verhältnis von 1A : 1B = 1 : 1, erhält man besonders hochchromatische Pigmentgemische mit sehr gutem Hautgefühl und mittlerem Deckvermögen.

### Beispiel 1c:

Ist das Verhältnis von 1A : 1B = 2 : 8, erhält man hochchromatische Pigmentgemische mit sehr hohem Deckvermögen und einem guten Hautgefühl.

### Beispiel 2: Pigmentgemisch mit roter Körperfarbe

### Ansatz 2A:

Es werden 8 g synthetische Glimmerplättchen der Fa. Merck KGaA (Teilchengröße < 15 µm) zusammen mit 80 g Siliziumdioxid-Kugeln der Fa. Sinoenergy (Durchmesser 2-4 µm) in 800 ml VE-Wasser gerührt. Diese Suspension wird unter Rühren auf die Reaktionstemperatur von 73 °C erhitzt und dann der pH-Wert mit 10 %iger Salzsäure auf 3,3 eingestellt. Anschließend werden 923 g 10 %ige Eisenchlorid-Lösung zudosiert, wobei der pH-Wert mit 20 %iger Natronlauge konstant gehalten wird. Nach vollständiger Zugabe wird 5 Minuten nachgerührt. Nun wird der pH-Wert mit 10 %iger HCl auf pH 2,1 gestellt, anschließend werden 310 g 25 %ige Titantetrachlorid-Lösung zu dosiert. Während dessen wird der pH-Wert mit 20 % iger Natronlauge konstant gehalten. Nach vollständiger Zugabe wird 5 Minuten nachgerührt. Danach wird der pH-Wert mit 20 %iger Natronlauge auf pH 7 gestellt, 15 min nachgerührt.

### Ansatz 2B:

1,7 l VE-Wasser werden unter Rühren auf die Reaktionstemperatur von 73 °C erhitzt und dann der pH-Wert mit 10 %iger Salzsäure auf 3,3 eingestellt. Anschließend werden 1000 g 10 %iger Eisenchlorid-Lösung zudosiert, wobei der pH-Wert mit 20 %iger Natronlauge konstant gehalten wird. Nach vollständiger Zugabe wird 5 Minuten nachgerührt und dann der pH-Wert mit 20 %iger Natronlauge auf pH 7 gestellt.

Nachdem beide Ansätze beendet sind, werden die Suspensionen der Ansätze 2A und 2B gemischt, abfiltriert, gewaschen und bei 150 °C getrocknet. Anschließend wird das Pigmentgemisch bei 780 °C geglüht und dann durch ein Sieb der Maschenweite 24 µm gegeben.

Man erhält ein Burgund-körperfarbenes Pigmentgemisch mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 2A/2B lassen sich Deckvermögen, Hautgefühl und Chroma beliebig einstellen.

### Beispiel 2a:

Ist das Verhältnis von 2A : 2B = 8 : 2, erhält man besonders hochchromatische Pigmentgemische mit außerordentlich gutem Hautgefühl und moderatem Deckvermögen.

### Beispiel 2b:

Ist das Verhältnis von 2A : 2B = 1 : 1, erhält man besonders hochchromatische Pigmentgemische mit sehr gutem Hautgefühl und mittlerem Deckvermögen.

### Beispiel 2c:

Ist das Verhältnis von 2A : 2B = 2 : 8, erhält man hochchromatische Pigmentgemische mit sehr hohem Deckvermögen und einem guten Hautgefühl.

### Beispiel 3: Pigmentgemisch mit weißer Körperfarbe

1,2 l VE-Wasser werden unter Rühren auf die Reaktionstemperatur von 75 °C erhitzt und dann der pH-Wert mit 10 %iger Salzsäure auf pH 1,8 eingestellt. Anschließend werden innerhalb von 25 Minuten 20 ml einer Titantetrachlorid-Lösung (400 g/l) zudosiert, wobei der pH-Wert mit 20 %iger Natronlauge konstant gehalten wird. Nach vollständiger Zugabe wird 20 Minuten nachgerührt und innerhalb von 30 Minuten 600 ml einer Suspension von 20 g natürliche Glimmerplättchen der Firma Merck KGaA (Teilchengröße < 15 µm) und 60 g Siliziumdioxid-Kugeln der Firma ABC Nanotech (Durchmesser 2-4 µm) in Wasser zudosiert, wobei der pH-Wert mit Salzsäure konstant gehalten wird. Anschließend werden innerhalb von 2,5 Stunden 200 ml einer Titantetrachlorid-Lösung (400 g/l) zudosiert, wobei der pH-Wert mit Natronlauge auf 1,8 gehalten wird. Nach einer Nachrührzeit wird der pH-Wert mit Natronlauge auf 5 gestellt und weitere 15 Minuten nachgerührt.

Der Ansatz wird abfiltriert, der Filterrückstand mit dem Produkt mit Wasser gewaschen und bei 150 °C getrocknet. Anschließend wird das Pulver 45 Minuten bei 800 °C geglüht und dann durch ein Sieb der Maschenweite 24 µm gesiebt.

Man erhält ein Pigmentgemisch mit weißer Körperfarbe, weichem Hautgefühl, matter Textur und edlem Schimmer, welches sich ohne weiteren Aufwand wie z.B. Aufmahlen, forciertes Dispergieren hervorragend in kosmetische Formulierungen einarbeiten lässt.

### Beispiel 4: Pigmentgemisch mit roter Körperfarbe

### Ansatz 4A:

Es werden 15 g natürliche Glimmerplättchen der Firma Merck KGaA (Teilchengröße < 15 µm) zusammen mit 85 g Siliziumdioxid-Kugeln der Fa. ABC Nanotech (Durchmesser 2-4 µm) in 1500 ml VE-Wasser gerührt. Diese Suspension wird unter Rühren auf die Reaktionstemperatur von 75 °C erhitzt. Der pH-Wert wird nun mit 10%iger Natronlauge auf pH 10 gestellt, dort für 5 Minuten gehalten und abschließend mit 10 %iger Salzsäure auf den Belegungs-pH 3,1 eingestellt. Anschließend werden 838 g 14 %ige Eisenchlorid-Lösung (170 g Fe₂O₃/l) zudosiert, wobei der pH-Wert während dessen mit 20 % iger Natronlauge konstant gehalten wird. Nach vollständiger Zugabe wird 15 Minuten nachgerührt. Nun wird der pH-Wert mit 10 %iger HCl auf pH 1,8 gestellt, anschließend 956 g Titantetrachlorid-Lösung, (400 g/l, was 133 g TiO₂ entspricht) zudosiert. Der pH-Wert wird mit 20 %iger Natronlauge konstant gehalten. Nach vollständiger Zugabe wird 5 Minuten nachgerührt. Danach wird der pH-Wert mit 10 %iger Natronlauge auf pH=5 gestellt und 15 Minuten nachgerührt.

Nach dem dieser Ansatz beendet ist, wird die Suspension abfiltriert, mit VE-Wasser gewaschen, bei 110 °C für 10 h getrocknet und 30 min bei 800 °C kalziniert.

Diese Vorstufe hat eine Zusammensetzung von 42 % Fe₂O₃, 33 % TiO₂, 21 % SiO₂ und 4% Glimmer.

### Ansatz 4B:

2 l VE-Wasser werden unter Rühren auf die Reaktionstemperatur von 85 °C erhitzt und dann der pH-Wert mit 10 %iger Salzsäure auf 2,8 eingestellt. Anschließend werden 295 g Eisenchlorid-Lösung (w(Fe)=14 %) zudosiert (entspricht 60 g Fe₂O₃), wobei der pH-Wert mit 20 %iger Natronlauge konstant gehalten wird. Nach vollständiger Zugabe wird 5 Minuten nachgerührt. Der Ansatz wird nicht direkt abgesaugt.

Anschließend werden 60 g des kalzinierten Materials aus Ansatz 4A zugegeben und dann der pH-Wert mit 20 %iger Natronlauge auf pH=5 gestellt und 15 Minuten nachgerührt. Die Suspension wird schließlich abfiltriert, mit 10 l VE-Wasser gewaschen und bei 110 °C für 10 h getrocknet. Abschließend wird das erhaltene Pulver 30 min bei 800 °C geglüht und mit einem 24 µm-Sieb gesiebt.

Das finale Produkt hat die folgende Zusammensetzung:
71 % Fe₂O₃, 16 % TiO₂, 11 % SiO₂ und 2% Glimmer.

Man erhält ein burgund-körperfarbenes Pigmentgemisch mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 4A/4B lassen sich Deckvermögen, Hautgefühl und Chroma beliebig einstellen.

### Beispiel 4a:

Ist das Verhältnis von 4A : 4B = 8 : 2, erhält man besonders hochchromatische Pigmentgemische mit außerordentlich gutem Hautgefühl und moderatem Deckvermögen.

### Beispiel 4b:

Ist das Verhältnis von 4A : 4B = 1 : 1, erhält man besonders hochchromatische Pigmentgemische mit sehr gutem Hautgefühl und mittlerem Deckvermögen.

### Beispiel 4c:

Ist das Verhältnis von 4A : 4B = 2 : 8, erhält man hoch-chromatische Pigmentgemische mit sehr hohem Deckvermögen und einem guten Hautgefühl.

### Beispiel 5: Pigmentgemisch mit champagnerfarbener Körperfarbe

### Ansatz 5A:

Es werden 8 g Aluminiumoxidplättchen der Firma Merck KGaA (5-40 µm) zusammen mit 80 g Siliziumdioxid-Kugeln der Fa. Sinoenergy (Durchmesser 2-4 µm) in 800ml VE-Wasser gerührt. Diese Suspension wird unter Rühren auf die Reaktionstemperatur von 73 °C erhitzt und dann der pH-Wert mit 10 %iger Salzsäure auf 2,1 eingestellt. Anschließend werden 2200 g einer 25%igen Titantetrachlorid-Lösung zudosiert. Der pH-Wert wird mit 20 %iger Natronlauge konstant gehalten. Nach vollständiger Zugabe wird 5 Minuten nachgerührt. Danach wird der pH-Wert mit 20 %iger Natronlauge auf pH 3,3 gestellt, und es wird 5 Minuten nachgerührt. Anschließend werden 356 g einer 7 %igen Eisenchlorid-Lösung zu dosiert, wobei der pH-Wert mit einer 20 %igen Natronlauge konstant gehalten wird. Nach vollständiger Zugabe der Lösung, wird 5 Minuten nachgerührt und dann der pH-Wert mit 20 %iger Natronlauge auf pH 6 gestellt.

### Ansatz 5B:

1,7 l VE-Wasser werden unter Rühren auf die Reaktionstemperatur von 70 °C erhitzt und dann der pH-Wert mit 10 %iger Salzsäure auf 2,1 eingestellt. Daraufhin werden 1350 g einer 25 %igen Titantetrachlorid-Lösung zudosiert. Der pH-Wert wird mit 20 %iger Natronlauge konstant gehalten. Danach wird der pH-Wert mit 20 %iger Natronlauge auf pH 3,3 eingestellt, und es wird 5 Minuten nachgerührt. Anschließend wird der pH-Wert auf 1,9 gestellt und es werden 356 g einer 7 %igen Eisenchlorid-Lösung zudosiert, wobei der pH-Wert mit einer 20 %igen Natronlauge konstant gehalten wird. Nach vollständiger Zugabe wird 5 Minuten nachgerührt. Danach wird der pH-Wert mit 20 %iger Natronlauge auf pH 6 gestellt.

Nachdem beide Ansätze beendet sind, werden die Suspensionen der Ansätze 5A und 5B gemischt, abfiltriert, gewaschen und bei 150 °C getrocknet. Anschließend wird das Pigmentgemisch bei 780 °C geglüht und dann durch ein Sieb der Maschenweite 24 µm gegeben.

Man erhält ein champagner- bis hautfarbenes Pigmentgemisch mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 5A/5B lassen sich Deckvermögen, Hautgefühl und Chroma beliebig einstellen.

### Beispiel 5a:

Ist das Verhältnis von 5A : 5B = 8 : 2, erhält man besonders hochchromatische Pigmentgemische mit außerordentlich gutem Hautgefühl und moderatem Deckvermögen.

### Beispiel 5b:

Ist das Verhältnis von 5A : 5B = 1 : 1, erhält man besonders hochchromatische Pigmentgemische mit sehr gutem Hautgefühl und mittlerem Deckvermögen.

### Beispiel 5c:

Ist das Verhältnis von 5A : 5B = 2 : 8, erhält man hochchromatische Pigmentgemische mit sehr hohem Deckvermögen und einem guten Hautgefühl.

### Beispiel 6: Pigmentgemisch mit champagnerfarbener Körperfarbe

### Ansatz 6A:

Es werden 12 g natürliche Glimmerplättchen der Firma Merck KGaA (<15 µm) zusammen mit 100 g Siliziumdioxid-Kugeln der Firma ABC Nanotech (Durchmesser 2-4 µm) in 800 ml VE-Wasser gerührt. Diese Suspension wird unter Rühren auf die Reaktionstemperatur von 85 °C erhitzt und dann der pH-Wert mit 10 %iger Salzsäure auf 2,1 eingestellt. Anschließend werden 1856 g einer 25 %igen Titantetrachlorid-Lösung zu dosiert. Der pH-Wert wird mit 20 %iger Natronlauge konstant gehalten. Nach vollständiger Zugabe wird 5 Minuten nachgerührt. Danach wird der pH-Wert mit 20 %iger Natronlauge auf pH 3,3 gestellt, und es wird 5 Minuten nachgerührt. Anschließend werden 289 g einer 7 %igen Eisenchlorid-Lösung zu dosiert, wobei der pH-Wert mit einer 20 %igen Natronlauge konstant gehalten wird. Nach vollständiger Zugabe der Lösung wird 5 Minuten nachgerührt und dann der pH-Wert mit 20%iger Natronlauge auf pH 6 gestellt. Die Suspension wird filtriert, salzfrei gewaschen und getrocknet. Das getrocknete Pigmentgemisch wird im Muffelofen bei 700 °C unter Luft kalziniert.

### Ansatz 6B:

1,7 l VE- Wasser werden unter Rühren auf die Reaktionstemperatur von 65 °C erhitzt und dann der pH-Wert mit 10 %iger Salzsäure auf 2,1 eingestellt. Daraufhin werden 1265 g einer 25 %igen Titantetrachlorid-Lösung zu dosiert. Der pH-Wert wird mit 20 %iger Natronlauge konstant gehalten. Danach wird der pH-Wert mit 20 %iger Natronlauge auf pH 3,1 eingestellt, und es wird 5 Minuten nachgerührt. Anschließend wird der pH-Wert auf 2,1 gestellt und es werden 564 g einer 7 % igen Eisenchlorid-Lösung zu dosiert, wobei der pH-Wert mit einer 20 % igen Natronlauge konstant gehalten wird. Nach vollständiger Zugabe wird 5 Minuten nachgerührt. Danach wird der pH-Wert mit 20 %iger Natronlauge auf pH 6 gestellt.

Nach dem beide Ansätze beendet sind, wird das kalzinierte Pulver aus Ansatz 6A zur Suspension von Ansatz 6B gegeben. Die Suspension wird filtriert, gewaschen und bei 150 °C getrocknet. Anschließend wird das Pulver bei 780 °C geglüht und dann durch ein Sieb der Maschenweite 24 µm gegeben.

Man erhält ein champagner- bis hautfarbenes Pigmentgemisch mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 6A/6B lassen sich Deckvermögen, Hautgefühl und Chroma beliebig einstellen.

### Beispiel 6a:

Ist das Verhältnis von 6A : 6B = 8 : 2, erhält man besonders hochchromatische Pigmentgemische mit außerordentlich gutem Hautgefühl und moderatem Deckvermögen.

### Beispiel 6b:

Ist das Verhältnis von 6A : 6B = 1 : 1, erhält man besonders hochchromatische Pigmentgemische mit sehr gutem Hautgefühl und mittlerem Deckvermögen.

### Beispiel 6c:

Ist das Verhältnis von 6A : 6B = 2 : 8, erhält man hoch-chromatische Pigmentgemische mit sehr hohem Deckvermögen und einem guten Hautgefühl.

### Beispiel 7: Pigmentgemisch mit champagnerfarbener Körperfarbe

### Ansatz 7A:

Es werden 15 g Aluminiumoxidplättchen der Firma Merck KGaA (< 20 µm) zusammen mit 85 g Siliziumdioxid-Kugeln der Firma ABC Nanotech (Durchmesser 2-4 µm) in 800 ml VE-Wasser gerührt.

Diese Suspension wird unter Rühren auf die Reaktionstemperatur von 73 °C erhitzt und dann der pH-Wert mit 10 %iger Salzsäure auf 3,1 eingestellt. Anschließend werden 356 g einer 7% igen Eisenchlorid-Lösung zu dosiert, wobei der pH-Wert mit einer 20 %igen Natronlauge konstant gehalten wird. Nach vollständiger Zugabe wird 5 Minuten nachgerührt. Danach wird der pH-Wert mit 20 %iger Salzsäure auf pH 2,1 eingestellt und 5 Minuten nachgerührt. Anschließend werden 1689 g einer 25 %igen Titantetrachlorid-Lösung zudosiert. Der pH-Wert wird mit 20 %iger Natronlauge konstant gehalten. Nach vollständiger Zugabe der Lösung, wird 5 Minuten nachgerührt und dann der pH-Wert mit 20 %iger Natronlauge auf pH 6 gestellt. Die Suspension wird filtriert, salzfrei gewaschen und bei 140 °C getrocknet.

### Ansatz 7B:

1,7 l VE-Wasser werden unter Rühren auf die Reaktionstemperatur von 85 °C erhitzt und dann der pH-Wert mit 10 %iger Salzsäure auf 3,1 eingestellt. Anschließend werden 685 g einer 7 %igen Eisenchlorid-Lösung zudosiert, wobei der pH-Wert mit einer 20 %igen Natronlauge konstant gehalten wird. Nach vollständiger Zugabe wird 5 Minuten nachgerührt. Daraufhin wird der pH-Wert mit 10 %iger Salzsäure auf pH 2,1 eingestellt und es werden 941 g einer 25 %igen Titantetrachlorid-Lösung zudosiert. Der pH-Wert wird mit 20 %iger Natronlauge konstant gehalten. Danach wird 5 Minuten nachgerührt und der pH-Wert mit 20 %iger Natronlauge auf pH 5 eingestellt.

Nach dem beide Ansätze beendet sind, wird das kalzinierte Pulver aus Ansatz A zur Suspension von Ansatz B gegeben. Die Suspension wird filtriert, gewaschen und bei 150 °C getrocknet. Anschließend wird das Pulver bei 550 °C geglüht und dann durch ein Sieb der Maschenweite 24 µm gegeben.

Man erhält ein champagner- bis hautfarbenes Pigmentgemisch mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 7A/7B lassen sich Deckvermögen, Hautgefühl und Chroma beliebig einstellen.

### Beispiel 7a:

Ist das Verhältnis von 7A : 7B = 8 : 2, erhält man besonders hochchromatische Pigmentgemische mit außerordentlich gutem Hautgefühl und moderatem Deckvermögen.

### Beispiel 7b:

Ist das Verhältnis von 7A : 7B = 1 : 1, erhält man besonders hochchromatische Pigmentgemische mit sehr gutem Hautgefühl und mittlerem Deckvermögen.

### Beispiel 7c:

Ist das Verhältnis von 7A : 7B = 2 : 8, erhält man hoch-chromatische Pigmentgemische mit sehr hohem Deckvermögen und einem guten Hautgefühl.

### Beispiel 8: Pigmentgemisch mit schwarzer Körperfarbe

### Ansatz 8A:

Es werden 8 g synthetische Siliziumdioxidplättchen der Firma Merck KGaA (5-50 µm) zusammen mit 80 g Siliziumdioxid-Kugeln der Firma ABC Nanotech (Durchmesser 2-4 µm) in 800 ml VE-Wasser gerührt.

Diese Suspension wird unter Rühren auf die Reaktionstemperatur von 73 °C erhitzt und dann der pH-Wert mit 10 %iger Salzsäure auf 3,3 eingestellt. Anschließend werden 923 g 10 %ige Eisenchlorid-Lösung zu dosiert, wobei der pH-Wert mit 20 %iger Natronlauge konstant gehalten wird. Nach vollständiger Zugabe wird 5 Minuten nachgerührt. Nun wird der pH-Wert mit 10 %iger HCl auf pH 2,1 gestellt, anschließend 310 g Titantetrachlorid-Lösung (400 g/l) zudosiert. Während dessen wird der pH-Wert mit 20 %iger Natronlauge konstant gehalten. Nach vollständiger Zugabe wird 5 Minuten nachgerührt. Danach wird der pH-Wert mit 20 %iger Natronlauge auf pH 7 gestellt, und es wird 15 Minuten nachgerührt.

### Ansatz 8B:

1,7 l VE-Wasser werden unter Rühren auf die Reaktionstemperatur von 73 °C erhitzt und dann der pH-Wert mit 10 %iger Salzsäure auf 3,3 eingestellt. Anschließend werden 1000 g 10 %iger Eisenchlorid-Lösung zu dosiert, wobei der pH-Wert mit 20 %iger Natronlauge konstant gehalten wird. Nach vollständiger Zugabe wird 5 Minuten nachgerührt und dann der pH-Wert mit 20 %iger Natronlauge auf pH 7 gestellt.

Nach dem beide Ansätze beendet sind, werden die Suspensionen der Ansätze 8A und 8B gemischt, abfiltriert, gewaschen und bei 150 °C getrocknet. Anschließend wird das Pulver bei 650 °C unter Formiergas (Verhältnis N₂ : H₂ = 95 : 5) kalziniert und dann durch ein Sieb der Maschenweite 24 µm gegeben.
Man erhält ein schwarzes körperfarbenes Effektpigment mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 8A/8B lassen sich Deckvermögen, Hautgefühl und Chroma beliebig einstellen.

### Beispiel 8a:

Ist das Verhältnis von 8A : 8B = 8 : 2, erhält man besonders hochchromatische Pigmentgemische mit außerordentlich gutem Hautgefühl und moderatem Deckvermögen.

### Beispiel 8b:

Ist das Verhältnis von 8A : 8B = 1 : 1, erhält man besonders hochchromatische Pigmentgemische mit sehr gutem Hautgefühl und mittlerem Deckvermögen.

### Beispiel 8c:

Ist das Verhältnis von 8A : 8B = 2 : 8, erhält man hochchromatische Pigmentgemische mit sehr hohem Deckvermögen und einem guten Hautgefühl.

### Beispiel 9: Pigmentgemisch mit schwarzer Körperfarbe

### Ansatz 9A:

Es werden 15 g natürliche Glimmerplättchen der Firma Merck KGaA (< 15 µm) zusammen mit 85 g Siliziumdioxid-Kugeln der Firma ABC Nanotech (Durchmesser 2-4 µm) in 1200 ml VE-Wasser gerührt.

Diese Suspension wird unter Rühren auf die Reaktionstemperatur von 80 °C erhitzt und dann der pH-Wert mit 10 %iger Salzsäure auf 3,1 eingestellt. Anschließend werden 886 g 14%ige Eisenchlorid-Lösung zudosiert, wobei der pH-Wert mit 32 %iger Natronlauge konstant gehalten wird. Nach vollständiger Zugabe wird 5 Minuten nachgerührt. Nun wird der pH-Wert mit 10 %iger HCl auf pH 2,1 gestellt, anschließend 1240 g Titantetrachlorid-Lösung (400 g/l) zudosiert. Der pH-Wert wird mit 20 %iger Natronlauge konstant gehalten. Nach vollständiger Zugabe wird 5 Minuten nachgerührt. Danach wird der pH-Wert mit 20 %iger Natronlauge auf pH 7 gestellt, und es wird 15 Minuten nachgerührt. Die Suspension wird filtriert, salzfrei gewaschen und getrocknet. Das getrocknete Pulver wird im Muffelofen bei 800 °C unter Luft kalziniert.

### Ansatz 9B:

1,7 l VE-Wasser werden unter Rühren auf die Reaktionstemperatur von 82 °C erhitzt und dann der pH-Wert mit 10 %iger Salzsäure auf 3,2 eingestellt. Anschließend werden 495 g 14% iger Eisenchlorid-Lösung zu dosiert, wobei der pH-Wert mit 20 %iger Natronlauge konstant gehalten wird. Nach vollständiger Zugabe wird 5 Minuten nachgerührt und dann der pH-Wert mit 20 %iger Natronlauge auf pH 5 gestellt.

Nachdem beide Ansätze beendet sind, wird das kalzinierte Pulver aus Ansatz A zur Suspension von Ansatz B gegeben. Die Suspension wird filtriert, gewaschen und bei 150 °C getrocknet. Anschließend wird das vereinigte Pulver bei 500 °C unter Formiergas (Verhältnis N₂ : H₂ = 92 : 8) kalziniert und dann durch ein Sieb der Maschenweite 24 µm gegeben. Man erhält ein schwarzes körperfarbenes Effektpigment mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 9A/9B lassen sich Deckvermögen, Hautgefühl und Chroma beliebig einstellen.

### Beispiel 9a:

Ist das Verhältnis von 9A : 9B = 8 : 2, erhält man besonders hochchromatische Pigmentgemische mit außerordentlich gutem Hautgefühl und moderatem Deckvermögen.

### Beispiel 9b:

Ist das Verhältnis von 9A : 9B = 1 : 1, erhält man besonders hochchromatische Pigmentgemische mit sehr gutem Hautgefühl und mittlerem Deckvermögen.

### Beispiel 9c:

Ist das Verhältnis von 9A : 9B = 2 : 8, erhält man hochchromatische Pigmentgemische mit sehr hohem Deckvermögen und einem guten Hautgefühl.

### Beispiel 10: Pigmentgemisch mit schwarzer Körperfarbe

### Ansatz 10A:

Es werden 10 g natürliche Glimmerplättchen der Firma Merck KGaA (10-60 µm) zusammen mit 85 g Siliziumdioxid-Kugeln der Fa. Sinoenergy (Durchmesser 2-4 µm) in 1200 ml VE-Wasser gerührt.

Diese Suspension wird unter Rühren auf die Reaktionstemperatur von 85 °C erhitzt und dann der pH-Wert mit 10 %iger Salzsäure auf 2,9 eingestellt. Anschließend werden 925 g 14 %ige Eisenchlorid-Lösung zu dosiert, wobei der pH-Wert mit 32 %iger Natronlauge konstant gehalten wird. Nach vollständiger Zugabe wird 5 Minuten nachgerührt. Nun wird der pH-Wert mit 10 %iger HCl auf pH 2,1 gestellt, anschließend 1015 g Titantetrachlorid-Lösung mit 400 g/l zudosiert. Während dessen wird der pH-Wert mit 20 %iger Natronlauge konstant gehalten. Nach vollständiger Zugabe wird 5 Minuten nachgerührt. Danach wird der pH-Wert mit 20 %iger Natronlauge auf pH 7 gestellt, und es wird 15 Minuten nachgerührt. Die Suspension wird filtriert, salzfrei gewaschen und getrocknet.

### Ansatz 10B:

1,7 l VE-Wasser werden unter Rühren auf die Reaktionstemperatur von 70 °C erhitzt und dann der pH-Wert mit 10 %iger Salzsäure auf 3,2 eingestellt. Anschließend werden 750 g 14 %iger Eisenchlorid-Lösung zu dosiert, wobei der pH-Wert mit 20 %iger Natronlauge konstant gehalten wird. Nach vollständiger Zugabe wird 5 Minuten nachgerührt und dann der pH-Wert mit 20 %iger Natronlauge auf pH 5 gestellt.

Nachdem beide Ansätze beendet sind, wird das getrocknete Pulver aus Ansatz A zur Suspension von Ansatz B gegeben. Die Suspension wird filtriert, gewaschen und bei 150 °C getrocknet. Anschließend wird das vereinigte Pulver bei 760 °C unter Formiergas (Verhältnis N₂ : H₂ = 92 : 8) kalziniert und durch ein Sieb der Maschenweite 24 µm gegeben.

Man erhält ein schwarzes körperfarbenes Effektpigment mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 10A/10B lassen sich Deckvermögen, Hautgefühl und Chroma beliebig einstellen.

### Beispiel 10a:

Ist das Verhältnis von 10A : 10B = 8 : 2, erhält man besonders hochchromatische Pigmentgemische mit außerordentlich gutem Hautgefühl und moderatem Deckvermögen.

### Beispiel 10b:

Ist das Verhältnis von 10A : 10B = 1 : 1, erhält man besonders hochchromatische Pigmentgemische mit sehr gutem Hautgefühl und mittlerem Deckvermögen.

### Beispiel 10c:

Ist das Verhältnis von 10A : 10B = 2 : 8, erhält man hochchromatische Pigmentgemische mit sehr hohem Deckvermögen und einem guten Hautgefühl.

### Beispiel 11: Pigmentgemisch mit roter Körperfarbe

### Ansatz 11A:

Es werden 15 g natürliche Glimmerplättchen der Firma Merck KGaA (5-35 µm) zusammen mit 85 g Siliziumdioxid-Kugeln der Firma ABC Nanotech (Durchmesser 2-4 µm) in 1200 ml VE-Wasser gerührt.

Diese Suspension wird unter Rühren auf die Reaktionstemperatur von 80 °C erhitzt und dann der pH-Wert mit 10 %iger Salzsäure auf 3,1 eingestellt. Anschließend werden 886 g 14 %ige Eisenchlorid-Lösung zu dosiert, wobei der pH-Wert mit 32 %iger Natronlauge konstant gehalten wird. Nach vollständiger Zugabe wird 5 Minuten nachgerührt. Nun wird der pH-Wert mit 10 %iger HCl auf pH 2,1 gestellt, anschließend 1240 g Titantetrachlorid-Lösung mit 400 g/l zu dosiert. Während dessen wird der pH-Wert mit 20 %iger Natronlauge konstant gehalten. Nach vollständiger Zugabe wird 5 Minuten nachgerührt. Danach wird der pH-Wert mit 20 %iger Natronlauge auf pH 7 gestellt, und es wird 15 Minuten nachgerührt. Die Suspension wird abfiltriert, salzfrei gewaschen und getrocknet. Das getrocknete Pulver wird im Muffelofen 30 Minuten bei 800 °C kalziniert.

### Ansatz 11B:

1,7 l VE-Wasser werden unter Rühren auf die Reaktionstemperatur von 82 °C erhitzt und dann der pH-Wert mit 10 %iger Salzsäure auf 3,2 eingestellt. Anschließend werden 495 g 14 %iger Eisenchlorid-Lösung zudosiert, wobei der pH-Wert mit 20 %iger Natronlauge konstant gehalten wird. Nach vollständiger Zugabe wird 5 Minuten nachgerührt und dann der pH-Wert mit 20 %iger Natronlauge auf pH 5 gestellt.

Nachdem beide Ansätze beendet sind, wird das kalzinierte Pulver aus Ansatz 11A zur Suspension von Ansatz 11B gegeben. Die Suspension wird filtriert, gewaschen und bei 150 °C getrocknet. Anschließend wird das vereinigte Pulver bei 780 °C geglüht und dann durch ein Sieb der Maschenweite 24 µm gegeben.
Man erhält ein burgund-körperfarbenes Effektpigment mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 11A : 11B lassen sich Deckvermögen, Hautgefühl und Chroma beliebig einstellen.

### Beispiel 11a:

Ist das Verhältnis von 11A : 11B = 8 : 2, erhält man besonders hochchromatische Pigmentgemische mit außerordentlich gutem Hautgefühl und moderatem Deckvermögen.

### Beispiel 11b:

Ist das Verhältnis von 11A : 11B = 1 : 1, erhält man besonders hochchromatische Pigmentgemische mit sehr gutem Hautgefühl und mittlerem Deckvermögen.

### Beispiel 11c:

Ist das Verhältnis von 11A : 11B = 2 : 8, erhält man hochchromatische Pigmentgemische mit sehr hohem Deckvermögen und einem guten Hautgefühl.

### Beispiel 12: Pigmentgemisch mit roter Körperfarbe

### Ansatz 12A:

Es werden 10 g synthetische Glimmerplättchen der Firma Merck KGaA (< 15 µm) zusammen mit 85 g Siliziumdioxid-Kugeln der Fa. Sinoenergy (Durchmesser 2-4 µm) in 1200 ml VE-Wasser gerührt.

Diese Suspension wird unter Rühren auf die Reaktionstemperatur von 85 °C erhitzt und dann der pH-Wert mit 10 %iger Salzsäure auf 2,9 eingestellt. Anschließend werden 925 g 14 %ige Eisenchlorid-Lösung zu dosiert, wobei der pH-Wert mit 32 %iger Natronlauge konstant gehalten wird. Nach vollständiger Zugabe wird 5 Minuten nachgerührt. Nun wird der pH-Wert mit 10%iger HCl auf pH 2,1 gestellt, anschließend 1015 g Titantetrachlorid-Lösung mit 400 g/l zu dosiert. Während dessen wird der pH-Wert mit 20 %iger Natronlauge konstant gehalten. Nach vollständiger Zugabe wird 5 Minuten nachgerührt. Danach wird der pH-Wert mit 20 %iger Natronlauge auf pH 7 gestellt, und es wird 15 Minuten nachgerührt. Die Suspension wird abfiltriert, salzfrei gewaschen, und getrocknet.

### Ansatz 12B:

1,7 l VE-Wasser werden unter Rühren auf die Reaktionstemperatur von 70 °C erhitzt und dann der pH-Wert mit 10 %iger Salzsäure auf 3,2 eingestellt. Anschließend werden 750 g 14 %iger Eisenchlorid-Lösung zudosiert, wobei der pH-Wert mit 20 %iger Natronlauge konstant gehalten wird. Nach vollständiger Zugabe wird 5 Minuten nachgerührt und dann der pH-Wert mit 20 %iger Natronlauge auf pH 5 gestellt.

Nachdem beide Ansätze beendet sind, wird das getrocknete Pulver aus Ansatz 12A zur Suspension von Ansatz 12B gegeben. Die Suspension wird filtriert, gewaschen und bei 150 °C getrocknet. Anschließend wird das vereinigte Pulver bei 780 °C geglüht und durch ein Sieb der Maschenweite 24 µm gegeben.

Man erhält ein burgund-körperfarbenes Pigmentgemisch mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 12A/12B lassen sich Deckvermögen, Hautgefühl und Chroma beliebig einstellen.

### Beispiel 12a:

Ist das Verhältnis von 12A : 12B = 8 : 2, erhält man besonders hochchromatische Pigmentgemische mit außerordentlich gutem Hautgefühl und moderatem Deckvermögen.

### Beispiel 12b:

Ist das Verhältnis von 12A : 12B = 1 : 1, erhält man besonders hochchromatische Pigmentgemische mit sehr gutem Hautgefühl und mittlerem Deckvermögen.

### Beispiel 12c:

Ist das Verhältnis von 12A : 12B = 2 : 8, erhält man hochchromatische Pigmentgemische mit sehr hohem Deckvermögen und einem guten Hautgefühl.

### Beispiel 13: Pigmentgemisch mit gelber Körperfarbe

### Ansatz 13A:

Es werden 8 g synthetische Glimmerplättchen der Firma Merck KGaA (10-40 µm) zusammen mit 80 g Siliziumdioxid-Kugeln der Firma ABC Nanotech (Durchmesser 2-4 µm) in 800 ml VE-Wasser gerührt.

Diese Suspension wird unter Rühren auf die Reaktionstemperatur von 85 °C erhitzt und dann der pH-Wert mit 10 %iger Schwefelsäure auf 3,5 eingestellt. Anschließend wird eine Lösung, bestehend aus 101 g Eisen(III)sulfat, 130 g Eisen(II)sulfat Heptahydrat und 480 g VE-Wasser zu dosiert, wobei der pH-Wert mit 20 %iger Natronlauge konstant gehalten wird. Nach vollständiger Zugabe wird 5 Minuten nachgerührt. Nun wird der pH-Wert mit 20 %iger Natronlauge auf pH 8 gestellt, worauf 150 g einer Natronwasserglas-Lösung (w SiO₂ = 14 %) zudosiert wird. Während dessen wird der pH-Wert mit 20 %iger Schwefelsäure konstant gehalten. Nach vollständiger Zugabe wird 5 Minuten nachgerührt. Danach wird der pH-Wert mit 10 %iger Schwefelsäure auf pH 5 gestellt, 15 min nachgerührt.

### Ansatz 13B:

1,7 l VE-Wasser werden unter Rühren auf die Reaktionstemperatur von 87 °C erhitzt und dann der pH-Wert mit 10 %iger Salzsäure auf 3,7 eingestellt. Anschließend wird eine Lösung bestehend aus 190 g Eisen(III)sulfat, 210 g Eisen(II)sulfat Heptahydrat und 600 g VE-Wasser zu dosiert, wobei der pH-Wert mit 20 %iger Natronlauge konstant gehalten wird. Nach vollständiger Zugabe wird 5 Minuten nachgerührt und dann der pH-Wert mit 20 %iger Natronlauge auf pH 7 gestellt, worauf 90 g einer Natronwasserglas-Lösung (w SiO₂ = 14 %) zudosiert wird. Während dessen wird der pH-Wert mit 20 %iger Schwefelsäure konstant gehalten. Nach vollständiger Zugabe wird 5 Minuten nachgerührt. Danach wird der pH-Wert mit 10 %iger Schwefelsäure auf pH 5 gestellt, 15 min nachgerührt.

Nach dem beide Ansätze beendet sind, werden die Suspensionen der Ansätze 13A und 13B gemischt, abfiltriert, gewaschen und bei 150 °C getrocknet und durch ein Sieb der Maschenweite 24 µm gegeben.

Man erhält ein gelbes körperfarbenes Pigmentgemisch mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 13A/13B lassen sich Deckvermögen, Hautgefühl und Chroma beliebig einstellen.

### Beispiel 13a:

Ist das Verhältnis von 13A : 13B = 8 : 2, erhält man besonders hochchromatische Pigmentgemische mit außerordentlich gutem Hautgefühl und moderatem Deckvermögen.

### Beispiel 13b:

Ist das Verhältnis von 13A : 13B = 1 : 1, erhält man besonders hochchromatische Pigmentgemische mit sehr gutem Hautgefühl und mittlerem Deckvermögen.

### Beispiel 13c:

Ist das Verhältnis von 13A : 13B = 2 : 8 erhält man hoch-chromatische Pigmentgemische mit sehr hohem Deckvermögen und einem guten Hautgefühl.

### Beispiel 14: Pigmentgemisch mit gelber Körperfarbe

### Ansatz 14A:

Es werden 15 g synthetische Glimmerplättchen der Firma Merck KGaA (5-40 µm) zusammen mit 85 g Siliziumdioxid-Kugeln der Fa. Sinoenergy (Durchmesser 2-4 µm) in 1200 ml VE-Wasser gerührt.

Diese Suspension wird unter Rühren auf die Reaktionstemperatur von 90 °C erhitzt und dann der pH-Wert mit 10 %iger Schwefelsäure auf 3,5 eingestellt. Anschließend wird eine Lösung, bestehend aus 200 g Eisen(III)sulfat, 210 g Eisen(II)sulfat Heptahydrat und 700 g VE-Wasser zu dosiert, wobei der pH-Wert mit 20 %iger Natronlauge konstant gehalten wird. Nach vollständiger Zugabe wird 5 Minuten nachgerührt. Nun wird der pH-Wert mit 20 %iger Natronlauge auf pH 8 gestellt, worauf 290 g einer Natronwasserglas-Lösung (w SiO₂ = 14 %) zudosiert wird. Während dessen wird der pH-Wert mit 20 %iger Schwefelsäure konstant gehalten. Nach vollständiger Zugabe wird 5 Minuten nachgerührt. Danach wird der pH-Wert mit 10%iger Schwefelsäure auf pH 5 gestellt, 15 min nachgerührt. Die Suspension wird filtriert, salzfrei gewaschen und getrocknet.

### Ansatz 14B:

1,2 l VE-Wasser werden unter Rühren auf die Reaktionstemperatur von 75 °C erhitzt und dann der pH-Wert mit 10 %iger Salzsäure auf 3,9 eingestellt. Anschließend wird eine Lösung, bestehend aus 120 g Eisen(III)sulfat, 163 g Eisen(II)sulfat Heptahydrat und 500 g VE-Wasser zu dosiert, wobei der pH-Wert mit 20 %iger Natronlauge konstant gehalten wird. Nach vollständiger Zugabe wird 5 Minuten nachgerührt und dann der pH-Wert mit 20 %iger Natronlauge auf pH 7 gestellt, worauf 65 g einer Natronwasserglas-Lösung (w SiO₂ = 14 %) zudosiert werden. Während dessen wird der pH-Wert mit 20 %iger Schwefelsäure konstant gehalten. Nach vollständiger Zugabe wird 5 Minuten nachgerührt. Danach wird der pH-Wert mit 10 %iger Schwefelsäure auf pH 6 gestellt, 15 min nachgerührt.

Nach dem beide Ansätze beendet sind, wird das getrocknete Pulver aus Ansatz 14A zur Suspension von Ansatz 14B gegeben. Die Suspension wird filtriert, gewaschen und bei 170 °C getrocknet und durch ein Sieb der Maschenweite 24 µm gegeben
Man erhält ein gelbes körperfarbenes Pigmentgemisch mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 14A/14B lassen sich Deckvermögen, Hautgefühl und Chroma beliebig einstellen.

### Beispiel 14a:

Ist das Verhältnis von 14A : 14B = 8 : 2, erhält man besonders hochchromatische Pigmentgemische mit außerordentlich gutem Hautgefühl und moderatem Deckvermögen.

### Beispiel 14b:

Ist das Verhältnis von 14A : 14B = 1 : 1, erhält man besonders hochchromatische Pigmentgemische mit sehr gutem Hautgefühl und mittlerem Deckvermögen.

### Beispiel 14c:

Ist das Verhältnis von 14A : 14B = 2 : 8, erhält man hochchromatische Pigmentgemische mit sehr hohem Deckvermögen und einem guten Hautgefühl.

### Beispiel 15: Pigmentgemisch mit grüner Körperfarbe

### Ansatz 15A:

Es werden 8 g natürliche Glimmerplättchen der Firma Merck KGaA (Teilchengröße 10-100 µm) zusammen mit 80 g Siliziumdioxid-Kugeln der Firma ABC Nanotech (Durchmesser 2-4 µm) in 1200 ml VE-Wasser gerührt.

Diese Suspension wird unter Rühren auf die Reaktionstemperatur von 70 °C erhitzt und dann der pH-Wert mit 10 %iger Salzsäure auf 5,5 eingestellt. Anschließend wird eine Lösung aus 309 g Chrom-III-Chlorid Hexahydrat und 800 ml VE-Wasser zu dosiert, wobei der pH-Wert mit 20 %iger Natronlauge konstant gehalten wird. Nach vollständiger Zugabe wird 5 Minuten nachgerührt. Danach wird der pH-Wert mit 20 %iger Natronlauge auf pH 7 gestellt, 15 min nachgerührt.

### Ansatz 15B:

2,1 l VE-Wasser werden unter Rühren auf die Reaktionstemperatur von 73 °C erhitzt und dann der pH-Wert mit 10 %iger Salzsäure auf 6,0 eingestellt. Anschließend wird eine Lösung aus 556 g Chrom-III-Chlorid Hexahydrat und 1250 ml VE-Wasser zu dosiert, wobei der pH-Wert mit 20 %iger Natronlauge konstant gehalten wird. Nach vollständiger Zugabe wird 5 Minuten nachgerührt und dann der pH-Wert mit 20 %iger Natronlauge auf pH 7 gestellt.

Nach dem beide Ansätze beendet sind, werden die Suspensionen der Ansätze 15A und 15B gemischt, abfiltriert, gewaschen und bei 150 °C getrocknet. Anschließend wird das Pulver bei 800 °C unter Luft kalziniert und dann durch ein Sieb der Maschenweite 24 µm gesiebt.

Man erhält ein grünes körperfarbenes Pigmentgemisch mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 15A/15B lassen sich Deckvermögen, Hautgefühl und Chroma beliebig einstellen.

### Beispiel 15a:

Ist das Verhältnis von 15A : 15B = 8 : 2, erhält man besonders hochchromatische Pigmentgemische mit außerordentlich gutem Hautgefühl und moderatem Deckvermögen.

### Beispiel 15b:

Ist das Verhältnis von 15A : 15B = 1 : 1, erhält man besonders hochchromatische Pigmentgemische mit sehr gutem Hautgefühl und mittlerem Deckvermögen.

### Beispiel 15c:

Ist das Verhältnis von 15A : 15B = 2 : 8, erhält man hochchromatische Pigmentgemische mit sehr hohem Deckvermögen und einem guten Hautgefühl.

### Beispiel 16: Pigmentgemisch mit grüner Körperfarbe

### Ansatz 16A:

Es werden 15 g natürliche Glimmerplättchen der Firma Merck KGaA (Teilchengröße 5-25 µm) zusammen mit 85 g Siliziumdioxid-Kugeln der Fa. Sinoenergy (Durchmesser 2-4 µm) in 1800 ml VE-Wasser gerührt.

Diese Suspension wird unter Rühren auf die Reaktionstemperatur von 80 °C erhitzt und dann der pH-Wert mit 10 %iger Salzsäure auf 1,8 eingestellt. Anschließend werden 350 g Titantetrachlorid-Lösung (400 g/l) zu dosiert. Der pH-Wert wird mit 20 % iger Natronlauge konstant gehalten. Nach vollständiger Zugabe wird 5 Minuten nachgerührt. Danach wird der pH-Wert mit 20 %iger Natronlauge auf pH 7 gestellt, 15 min nachgerührt. Anschließend wird eine Lösung aus 516 g Chrom-III-Chlorid Hexahydrat und 1010 ml VE-Wasser zu dosiert, wobei der pH-Wert mit 20 %iger Natronlauge konstant gehalten wird. Nach vollständiger Zugabe wird 5 Minuten nachgerührt. Die Suspension wird filtriert, salzfrei gewaschen und getrocknet. Das getrocknete Pulver wird im Muffelofen bei 800 °C unter Luft kalziniert.

### Ansatz 16B:

1,9 l VE-Wasser werden unter Rühren auf die Reaktionstemperatur von 80 °C erhitzt und dann der pH-Wert mit 10 %iger Salzsäure auf 6,0 eingestellt. Anschließend wird eine Lösung aus 706 g Chrom-III-Chlorid Hexahydrat und 1200 ml VE-Wasser zu dosiert, wobei der pH-Wert mit 20 %iger Natronlauge konstant gehalten wird. Nach vollständiger Zugabe wird 5 Minuten nachgerührt und dann der pH-Wert mit 20 %iger Natronlauge auf pH 7 gestellt.
Nach dem beide Ansätze beendet sind, wird das kalzinierte Pulver aus Ansatz A zur Suspension von Ansatz B gegeben. Die Suspension wird filtriert, gewaschen und bei 150 °C getrocknet. Anschließend wird das vereinigte Pulver bei 750 °C unter Luft kalziniert und durch ein Sieb der Maschenweite 24 µm gegeben.

Man erhält ein grünes körperfarbenes Pigmentgemisch mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 16A/16B lassen sich Deckvermögen, Hautgefühl und Chroma beliebig einstellen.

### Beispiel 16a:

Ist das Verhältnis von 16A : 16B = 8 : 2 erhält man besonders hochchromatische Pigmentgemische mit außerordentlich gutem Hautgefühl und moderatem Deckvermögen.

### Beispiel 16b:

Ist das Verhältnis von 16A : 16B = 1 : 1 erhält man besonders hochchromatische Pigmentgemische mit sehr gutem Hautgefühl und mittlerem Deckvermögen.

### Beispiel 16c:

Ist das Verhältnis von 16A : 16B = 2 : 8, erhält man hochchromatische Pigmentgemische mit sehr hohem Deckvermögen und einem guten Hautgefühl.

### Beispiel 17: Pigmentgemisch mit grüner Körperfarbe

### Ansatz 17A:

Es werden 10 g natürliche Glimmerplättchen der Firma Merck KGaA (Teilchengröße < 15 µm) zusammen mit 100 g Siliziumdioxid-Kugeln der Firma ABC Nanotech (Durchmesser 2-4 µm) in 1800 ml VE-Wasser gerührt.

Diese Suspension wird unter Rühren auf die Reaktionstemperatur von 80 °C erhitzt und dann der pH-Wert mit 10 %iger Salzsäure auf 2,1 eingestellt. Anschließend werden 200 g Titantetrachlorid-Lösung (400 g/l) zudosiert. Der pH-Wert wird mit 20 %iger Natronlauge konstant gehalten. Nach vollständiger Zugabe wird 5 Minuten nachgerührt. Danach wird der pH-Wert mit 20 %iger Natronlauge auf pH 7,5 gestellt, 15 min nachgerührt. Anschließend wird eine Lösung aus 480 g Chrom-III-Chlorid Hexahydrat und 900 ml VE-Wasser zu dosiert, wobei der pH-Wert mit 20 %iger Natronlauge konstant gehalten wird. Nach vollständiger Zugabe wird 5 Minuten nachgerührt. Die Suspension wird filtriert, salzfrei gewaschen und getrocknet.

### Ansatz 17B:

1,9 l VE-Wasser werden unter Rühren auf die Reaktionstemperatur von 85 °C erhitzt und dann der pH-Wert mit 10 %iger Salzsäure auf 6,5 eingestellt. Anschließend wird eine Lösung aus 706 g Chrom-III-Chlorid Hexahydrat und 950 ml VE-Wasser zu dosiert, wobei der pH-Wert mit 20 %iger Natronlauge konstant gehalten wird. Nach vollständiger Zugabe wird 5 Minuten nachgerührt und dann der pH-Wert mit 20 %iger Natronlauge auf pH 7 eingestellt.

Nachdem beide Ansätze beendet sind, wird das getrocknete Pulver aus Ansatz 17A zur Suspension von Ansatz 17B gegeben. Die Suspension wird filtriert, gewaschen und bei 150 °C getrocknet. Anschließend wird das vereinigte Pulver bei 850 °C unter Luft kalziniert und durch ein Sieb der Maschenweite 24 µm gegeben.

Man erhält ein grünes körperfarbenes Pigmentgemisch mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 17A/17B lassen sich Deckvermögen, Hautgefühl und Chroma beliebig einstellen.

### Beispiel 17a:

Ist das Verhältnis von 17A : 17B = 8 : 2, erhält man besonders hochchromatische Pigmentgemische mit außerordentlich gutem Hautgefühl und moderatem Deckvermögen.

### Beispiel 17b:

Ist das Verhältnis von 17A : 17B = 1 : 1, erhält man besonders hochchromatische Pigmentgemische mit sehr gutem Hautgefühl und mittlerem Deckvermögen.

### Beispiel 17c:

Ist das Verhältnis von 17A : 17B = 2 : 8, erhält man hoch-chromatische Pigmentgemische mit sehr hohem Deckvermögen und einem guten Hautgefühl.

### Beispiel 18: Pigmentgemisch mit weißer Körperfarbe

### Ansatz 18A:

Es werden 20 g natürliche Glimmerplättchen der Firma Merck KGaA (Teilchengröße 10-60 µm) zusammen mit 65 g Siliziumdioxid-Kugeln der Fa. Sinoenergy (Durchmesser 2-4 µm) in 1200 ml VE-Wasser gerührt. Diese Suspension wird unter Rühren auf die Reaktionstemperatur von 75 °C erhitzt und dann der pH-Wert mit 10 %iger Salzsäure auf 2,0 eingestellt. Anschließend werden 150 g 10 %ige Zinn(IV)-chlorid-Lösung innerhalb 45 min zudosiert, wobei der pH-Wert mit 20 %iger Natronlauge konstant gehalten wird. Nach vollständiger Zugabe wird 5 Minuten nachgerührt. Danach werden 1300 g 12 %iger Titantetrachlorid-Lösung von 6 Stunden zudosiert. Der pH-Wert wird mit 20 %iger Natronlauge konstant gehalten. Nach vollständiger Zugabe wird 5 Minuten nachgerührt. Danach wird der pH-Wert mit 20 %iger Natronlauge auf pH 6 gestellt und 15 min nachgerührt.

### Ansatz 18B:

1,5 l VE-Wasser werden unter Rühren auf die Reaktionstemperatur von 75 °C erhitzt und dann der pH-Wert mit 10 %iger Salzsäure auf pH 3,0 eingestellt. Anschließend werden innerhalb 4 Stunden 1900 g 14 %iger Titantetrachlorid-Lösung zudosiert, wobei der pH-Wert mit 20 %iger Natronlauge konstant gehalten wird. Nach vollständiger Zugabe wird 5 Minuten nachgerührt und dann der pH-Wert mit 20 %iger Natronlauge auf pH 7 gestellt.
Nachdem beide Ansätze beendet sind, werden die Suspensionen der Ansätze 18A und 18B gemischt, abfiltriert, mit Wasser gewaschen und bei 150 °C getrocknet. Anschließend wird das Pulver 45 min bei 800 °C geglüht und dann durch ein Sieb mit Maschenweite 100 µm gesiebt.

Man erhält ein Pigmentgemisch mit weißer Körperfarbe, weichem Hautgefühl, matter Textur und edlem Schimmer, welches sich ohne weiteren Aufwand wie z.B. Aufmahlen, forciertes Dispergieren hervorragend in kosmetische Formulierungen einarbeiten lässt. Durch das Mischungsverhältnis von 18A/18B lassen sich Deckvermögen, Hautgefühl und Luminanz (Schimmer) beliebig einstellen.

### Beispiel 18a:

Ist das Verhältnis von 18A : 18B= 8 : 2, erhält man besonders hochchromatische Pigmentgemische mit außerordentlich gutem Hautgefühl und moderatem Deckvermögen.

### Beispiel 18b:

Ist das Verhältnis von 18A : 18B = 1 : 1, erhält man besonders hochchromatische Pigmentgemische mit sehr gutem Hautgefühl und mittlerem Deckvermögen.

### Beispiel 18c:

Ist das Verhältnis von 18A : 18B = 2 : 8, erhält man hochchromatische Pigmentgemische mit sehr hohem Deckvermögen und einem guten Hautgefühl.

### Beispiel 19: Pigmentgemisch mit blauer Körperfarbe

### Ansatz 19A:

Es werden 8 g Glasplättchen der Fa. Merck KGaA (Teilchengröße 10-100 µm) zusammen mit 80 g Siliziumdioxid-Kugeln der Firma ABC Nanotech, (Durchmesser: 2-4 µm) in 800 ml VE-Wasser gerührt.

Diese Suspension wird unter Rühren auf die Reaktionstemperatur von 75 °C erhitzt. Der pH-Wert wird mittels 10 %iger Schwefelsäure auf 2,1 eingestellt dann 2200 g einer 25 %igen Titantetrachlorid-Lösung zudosiert. Der pH-Wert wird mit 20 %iger Natronlauge konstant gehalten. Nach vollständiger Zugabe wird 5 Minuten nachgerührt und dann der pH-Wert mit 10 %iger Schwefelsäure auf 4,3 eingestellt. Anschließend werden gleichzeitig zwei Lösungen 1 und 2 zugeben, während der pH-Wert mit einer 10 %igen Ammoniumcarbonat-Lösung konstant gehalten wird. Lösung 1 wird aus 60 g Kaliumhexacyanoferrat(III) und 1300 g VE-Wasser hergestellt. Lösung 2 besteht aus 76 g Eisensulfat Heptahydrat und 1250 g VE-Wasser. Nach vollständiger Zugabe werden 15 min und dann der pH-Wert mit 20 %iger Natronlauge auf pH 6 gestellt.

### Ansatz 19B:

Es werden 900 ml VE-Wasser unter Rühren auf die Reaktionstemperatur von 73 °C erhitzt und dann der pH-Wert mit 10 %iger Schwefelsäure 4,3 eingestellt. Anschließend werden gleichzeitig zwei Lösungen 1 und 2 zugeben, während der pH-Wert mit einer 10 %igen Ammoniumcarbonat-Lösung konstant gehalten wird. Lösung 1 wird aus 165 g Kaliumhexacyanoferrat(III) und 2100 g VE-Wasser hergestellt. Lösung 2 besteht aus 209 g Eisensulfat Heptahydrat und 1950 g VE-Wasser. Danach wird der pH-Wert mit 20 %iger Natronlauge auf pH 6 gestellt.

Nachdem beide Ansätze beendet sind, werden die Suspensionen der Ansätze 1A und 1B gemischt, abfiltriert, gewaschen und bei 150 °C getrocknet und dann durch ein Sieb der Maschenweite 24 µm gesiebt.

Man erhält ein blaues körperfarbenes Pigmentgemisch mit weichem Hautgefühl und matter Textur, welches ein hohes Chroma aufweist und sich ohne weiteren Aufwand hervorragend in kosmetische Formulierungen einarbeiten lässt. Durch das Mischungsverhältnis von 19A/19B lassen sich Deckvermögen, Hautgefühl und Chroma beliebig einstellen.

### Beispiel 19a:

Ist das Verhältnis von 19A : 19B = 8 : 2, erhält man besonders hochchromatische Pigmentgemische mit außerordentlich gutem Hautgefühl und moderatem Deckvermögen.

### Beispiel 19b:

Ist das Verhältnis von 19A : 19B = 1 : 1, erhält man besonders hochchromatische Pigmentgemische mit sehr gutem Hautgefühl und mittlerem Deckvermögen.

### Beispiel 19c:

Ist das Verhältnis von 19A : 19B = 2 : 8,erhält man hoch-chromatische Pigmentgemische mit sehr hohem Deckvermögen und einem guten Hautgefühl.

### Beispiel 20: Pigmentgemisch mit weißer Körperfarbe

In einem temperierbaren Reaktionsgefäß werden 1500 ml entsalztes Wasser vorgelegt, auf 75°C erhitzt und bei dieser Temperatur 376 g TiOCl₂-Lösung (400 g TiCl₄/Liter) mit einer Dosierrate von 5 ml/min. unter intensivem Rühren zudosiert. Der pH-Wert sinkt dabei ab und wird durch kontrollierte Zugabe von 32 %iger Natronlauge bei 2,0 konstant gehalten. Nach Ende der TiOCl₂-Zugabe wird 15 Min. bei pH 2,0 nachgerührt. Anschließend wird eine homogene Suspension von 75 g Glimmerplättchen (Teilchengröße 1 - 15 µm) und 25 g SiO₂-Kugeln (D₅₀ = 2 - 4 µm ; SILNOS 130 der Fa. ABC Nanotech Co., Ltd.) in 250 ml entsalztem Wasser zugegeben und nach Wiedererreichen von 75 °C der pH-Wert mit Salzsäure (18 % HCl) auf 2,2 abgesenkt. Unter Konstanthaltung der Temperatur und des pH-Wertes mit 32 %iger Natronlauge wird nun eine homogene Lösung aus 8,64 g SnOCl₂-Lösung (50 % SnCl₄), 22,8 g Salzsäure (37 % HCl) und 167 g entsalztem Wasser innerhalb von 30 Min. gleichmäßig zudosiert. Nach einer Nachrührzeit von 15 Min. werden 740 g TiOCl₂-Lösung (400 g TiCl₄/Liter) mit einer Dosierrate von 5 ml/min. zugegeben. Dabei wird der pH-Wert mit 32 %iger Natronlauge bei 2,2 konstant gehalten. Nach Beendigung der Zugabe der TiOCl₂-Lösung wird 15 Min. nachgerührt, dann der pH-Wert mit 32 %iger Natronlauge auf pH 5,0 eingestellt und weitere 15 Min. nachgerührt.

Der erhaltene Feststoff wird abfiltriert, mit 15 l entsalztem Wasser gewaschen und 16 Stunden bei 110 °C getrocknet. Abschließend wird 30 Min. bei 850 °C geglüht und durch ein Sieb der Maschenweite 100 µm gesiebt.

Man erhält ein weißes Pulver mit sehr weichem Hautgefühl, das bei Ausstreichen auf der Haut einen leichten, bläulichen Glanz zeigt.

### Anwendungsbeispiele

### Beispiel A1: Eye Shadow Gel

| **Rohstoff** | | **INCI** | **[%]** |
|---|---|---|---|
| Phase A | | | |
| Timiron® Super Gold | (1) | GLIMMERPLÄTTCHEN DER FIRMA MERCK KGAA, CI 77891 (TITANIUM DIOXIDE) | 15,00 |
| Pigmentgemisch aus Beispiel 1 Carbopol Ultrez 21 | | | 7,00 |
| | (2) | ACRYLATES/C10-30 ALKYL ACRYLATE CROSSPOLYMER | 0,30 |
| Aloe Vera Powder regular 200x Citronensäure Monohydrat Wasser, demineralisiert | (3) | ALOE BARBADENSIS | 0,05 |
| | (1) | CITRIC ACID | 0,00 |
| | | AQUA (WATER) | 55,87 |

| Phase B | | | |
|---|---|---|---|
| Triethanolamin 90 % Care Germaben II | (4) | TRIETHANOLAMINE, AQUA | 0,78 |
| | (5) | (WATER) PROPYLENE GLYCOL, DIAZOLIDINYL UREA, METHYLPARABEN, PROPYLPARABEN | 1,00 |
| Glycerin, wasserfrei Wasser, demineralisiert Phase C Lubrajel DV | (1) | GLYCERIN | 2,00 |
| | | AQUA (WATER) | 13,00 |
| | (6) | PROPYLENE GLYCOL, POLYGLYCERYLMETHACRYLATE | 5,00 |

### Herstellung:

Aloe Vera Puder im Wasser der Phase A lösen, dann alle Pigmente und das Pigmentgemisch und die restlichen Inhaltsstoffe bis auf das Carbopol zufügen und dispergieren. Mit einigen Tropfen Citronensäure ansäuern, um die Viskosität zu vermindern, dann Carbopol unter Rühren einstreuen. Nach vollständiger Lösung die vorgelöste Phase B langsam einrühren (nicht homogenisieren) und anschließend Phase C zugeben. Falls erforderlich, den pH-Wert zwischen 7,0-7,5 mit Citronensäurelösung einstellen.

Man erhält eine wasserbasierende Eye Shadow Gel Formulierung mit Aloe Vera. (Schnellsttrocknend und gut mit den Fingern aufzutragen.)

### Bezugsquellen:

(1) Merck KGaA/Rona®
(2) Noveon
(3) Terry Laboratoires, Inc.
(4) BASF AG
(5) ISP Global Technologies
(6) Guardian

### Beispiel A2: Creamy Eyeshadow

| **Rohstoff** | | **INCI** | **[%]** |
|---|---|---|---|
| Phase A | | | |
| Colorona® Light Blue | (1) | GLIMMERPLÄTTCHEN DER FIRMA MERCK KGAA, CI 77891 (TITANIUM DIOXIDE) CI 77510 (FERRIC FERROCYANIDE) | 10,00 |
| **Pigmentgemisch aus Beispiel 1** | | | 15,00 |
| Talkum | (1) | TALC | 12,00 |

| Phase B | | | |
|---|---|---|---|
| Crodamol PMP | (2) | PPG-2MYRISTYL ETHER PROPIONATE | 32,80 |
| Miglyol 812 N | (3) | CAPRYLIC/CAPRIC TRIGLYCERIDE | 12,00 |
| Syncrowax HGLC | (2) | C18-36 ACID TRIGLYCERIDE | 10,00 |
| Syncrowax HRC | (2) | TRIBEHENIN | 3,00 |
| Parteck® LUB STA | (1) | STEARIC ACID | 3,00 |
| Antaron V-216 | (4) | PVP/HEXADECENE COPOLYMER | 2,00 |
| Oxynex® K flüssig | (1) | PEG-8, TOCOPHEROL, ASCORBYL PALMITATE, ASCORBIC ACID, CITRIC ACID | 0,10 |
| Propyl-4-hydroxybenzoat | (1) | PROPYLPARABEN | 0,10 |

### Herstellung:

Phase B auf ca. 80 °C erhitzen bis alles geschmolzen ist und unter Rühren auf 65 °C abkühlen. Unter Rühren nun die Inhaltsstoffe der Phase A zugeben und die Masse bei 65 °C in das vorgesehene Packmittel gießen. Auf Raumtemperatur abkühlen lassen.

### Bezugsquellen:

(1) Merck KGaA/Rona®
(2) Croda GmbH
(3) Sasol Germany GmbH
(4) ISP Global Technologies

### Beispiel A3: Gesichtspuder

| **Rohstoff** | | **INCI** | **[%]** |
|---|---|---|---|
| Phase A | | | |
| Pigmentgemisch aus Beispiel 5 | | | 20,00 |
| Unipure Yellow LC 182 | (1) | CI 77492 (IRON OXIDES) | 1,20 |
| Unipure Red LC 381 | (1) | CI 77491 (IRON OXIDES) | 0,20 |
| Unipure Brown LC 889 | (1) | CI 77491 (IRON OXIDES) CI 77499 (IRON OXIDES) | 0,30 |
| Magnesiumstearat | (2) | MAGNESIUM STEARATE | 2,00 |
| Talkum | (2) | TALC | 71,90 |

| Phase B | | | |
|---|---|---|---|
| RonaCare® all-rac-alpha-Tocopherylacetat | (2) | TOCOPHERYLACETATE | 0,30 |
| | | | |
| Parfümöl 200 529 Eutanol G | (3) | PARFUM | 0,30 |
| | (4) | OCTYLDODECANOL | 3,70 |
| Propyl-4-hydroxybenzoat | (2) | PROPYLPARABEN | 0,10 |
| Herstellung: | | | |

Die Bestandteile der Phase A in den Mixer (z. B. La Moulinette von Moulinex) geben und 2 x 10 Sekunden mixen. Die Mischung in ein Becherglas überführen, Phase B hinzugeben und mit dem Spatel vorab verrühren. Die Mischung aus Phase A und Phase B wiederum in den Mixer geben und 3 x 10 Sekunden zu einer homogenen Phase verarbeiten.

Der Pressdruck für eine Puderpfanne mit 36 mm Durchmesser beträgt ca. 25 bar.

### Bezugsquellen:

(1) Les Colorants Wackherr
(2) Merck KGaA/Rona®
(3) Fragrance Resources
(4) Cognis GmbH

### Beispiel A4: Mattifying Foundation

| **Rohstoff** | | **INCI** | **[%]** |
|---|---|---|---|
| Phase A | | | |
| Wasser, demineralisiert | | AQUA (WATER) | 57,89 |
| Pigmentgemisch aus Beispiel 13 | | | 6,00 |
| Glycerin (87 % reinst) | (1) | GLYCERIN, AQUA (WATER) | 5,00 |
| RonaCare® Ectoin | (1) | ECTOIN | 0,30 |
| Keltrol CG-SFT | (2) | XANTHAN GUM | 0,15 |
| Triethanolamin 90% Care | (3) | TRIETHANOLAMINE, AQUA (WATER) | 0,13 |

| Phase B | | | |
|---|---|---|---|
| Kronos 1001 | (4) | CI 77891 (TITANIUM DIOXIDE) | 4,92 |
| Unipure Yellow LC 182 | (5) | CI 77492 (IRON OXIDES) | 1,60 |
| Unipure Red LC 381 | (5) | CI 77491 (IRON OXIDES) | 0,20 |
| Unipure Brown LC | (5) | CI 77491 (IRON OXIDES) CI | 0,20 |
| 889 Unipure Blue LC 686 | (5) | 77499 (IRON OXIDES) CI 77007 (ULTRAMARIN BLUE) | 0,08 |

| Phase C | | | |
|---|---|---|---|
| Miglyol 812N | (6) | CAPRYLIC/CAPRIC TRIGLYCERIDE | 7,00 |
| Eutanol G | (7) | OCTYLDODECANOL | 4,00 |
| Montanov 202 | (8) | ARACHIDYL ALCOHOL, BEHENYL ALCOHOL, ARACHIDYLGLUCOSIDE | 4,00 |
| Avocadoöl | (9) | PERSEA GRATISSIMA (AVOCADO OIL) | 2,00 |
| Eusolex® 9020 | (1) | BUTYL METHOXYDIBENZOYLMET HANE | 1,50 |
| Hydrolite-5 | (10) | PENTYLENE GLYCOL | 1,20 |
| Bentone Gle GTCC V | (11) | STEARALKONIUM HECTORITE, PROPYLENE CARBONATE, CAPRYLIC/CAPRIC TRIGLYCERIDE | 1,00 |
| RonaCare® all-rac-alpha-Tocopherylacetat | (2) | TOCOPHERYLACETATE | 0,50 |
| Phenonip | (12) | PHENOXYETHANOL, BUTYLPARABEN , ETHYLPARABEN , PROPYLP ARABEN , METHYLPARABEN | 0,40 |
| Oxynex® K flüssig | (1) | PEG-8, TOCOPHEROL, ASCORBYL PALMITATE, ASCORBIC ACID, CITRIC ACID | 0,03 |

| Phase D | | | |
|---|---|---|---|
| Simulgel EG | (8) | SODIUM ACRYLATE/SODIUM ACRYLOYLDIMETHYL TAUR ATE COPOLYMER, ISOHEXADECANE, POLYSORBATE 80 | 0,60 |

| Phase E | | | |
|---|---|---|---|
| Wasser, demineralisiert | | AQUA (WATER) | 1,00 |

### Herstellung:

Keltrol langsam ins Wasser der Phase A geben und dispergieren. Restliche Bestandteile der Phase A unter Rühren einstreuen. Die Bestandteile der Phase B zur Phase A geben und mit dem Ultra-Turrax T25 (Stufe rot-blau, 13500-20500 Upm) 3 min homogenisieren und auf Agglomerate prüfen. Phase A/B und Phase C separat auf 75 °C erhitzen. Unter Rühren Phase C zu Phase A/B geben und 2 min mit dem Ultra-Turrax T25 (Stufe gelb-grün, 8000-9500 Upm) homogenisieren. Zwischen 55-60 °C Phase D zugeben, bei 40 °C Phase E zugeben, unter weiterem Rühren auf Raumtemperatur abkühlen; pH-Wert auf 7,0 mit 30 %iger Citronensäure einstellen. Dann in geeignete Behältnisse abfüllen.

Man erhält eine leichte, schwach deckende Foundation, die für alle Hauttypen geeignet ist. Avocadoöl, Vitamin-E-Acetat und das zellschützende RonaCare® Ectoin unterstützen die hautpflegende Wirkung.

### Bezugsquellen:

(1) Merck KGaA/Rona®
(2) C.P. Kelco
(3) BASF AG
(4) Kronos International Inc.
(5) Les Colorants Wackherr
(6) Sasol Germany GmbH
(7) Cognis GmbH
(8) Seppic
(9) Gustav Heess GmbH
(10) Symrise
(11) Elementis Specialities
(12) Clariant GmbH

### Beispiel A5: Body Lotion

| **Rohstoff** | | **INCI** | **[%]** |
|---|---|---|---|
| Phase A | | | |
| Aloe Vera Gel 10x declorized | (1) | ALOE BARBADENSIS | 2,00 |
| D-Panthenol | (2) | PANTHENOL | 0,40 |
| Pigmentgemisch aus Beispiel 3 | | | 6,00 |
| RonaCare® Allantoin | (3) | ALLANTOIN | 0,20 |
| Glycerin, wasserfrei | (3) | GLYCERIN | 4,00 |
| Wasser, demineralisiert | | AQUA (WATER) | 67,57 |

| Phase B | | | |
|---|---|---|---|
| Protelan AGL 95/C | (4) | SODIUM COCOYL GLUTAMATE | 6,00 |
| Cosmacol EMI | (5) | DI-C12-13 ALKYL MALATE | 3,00 |
| Eutanol G | (6) | OCTYLDODECANOL | 3,00 |
| Jojobaöl | (7) | SIMMONDSIA CHINENSIS (JOJOBA OIL) | 1,50 |
| Tegosoft TN | (8) | C12-15 ALKYL ALKYL BENZOATE | 1,50 |
| Carbopol ETD 2020 | (9) | ACRYLATES/C10-30 ALKYL ACRYLATE CROSSPOLYMER | 0,60 |
| Phenonip | (10) | PHENOXYETHANOL, BUTYLPARABEN , ETHYLPARABEN , PROPYLPARABEN, METHYLPARABEN | 0,60 |
| RonaCare® Bisabolol | (3) | BISABOLOL | 0,50 |
| RonaCare® all-racalpha-Tocopherylacetat | (3) | TOCOPHERYLACETATE | 0,50 |
| Oxynex® ST Liquid | (3) | DIETHYLHEXYL SYRINGYLIDENEMALONATE, CAPRYLIC/CAPRIC TRIGLYCERIDE | 0,50 |
| Cremophor RH 410 | (11) | PEG-40 HYDROGENATED CASTOR OIL | 0,30 |
| Oxynex® K flüssig | | PEG-8, TOCOPHEROL, ASCORBYL PALMITATE, ASCORBIC ACID, CITRIC ACID | 0,03 |

| Phase C | | | |
|---|---|---|---|
| Parfümöl Lifetime DH10255/1 Phase D | (12) | PARFUM | 0,50 |
| Wasser, demineralisiert | | AQUA (WATER) | 1,00 |
| Germal 115 | (13) | IMIDAZOLIDINYL UREA | 0,30 |

### Herstellung:

Aloe Vera und RonaCare® Allantoin im Wasser der Phase A unter Rühren vorlösen, dann die anderen Bestandteile der Phase A zugeben und auf 60 °C erhitzen, Jojobaöl, Oxynex K flüssig, Cosmacol EMI, Eutanol G und Tegosoft TN in einem Rührgefäß vorlegen, dann Carbopol mit der Dispergierscheibe (ca. 700 U/min, 20 min) dazu homogen einarbeiten. Dann die restlichen Bestandteile der Phase B zufügen und alles zu einer homogenen Mischung verrühren, wobei Protelan AGL 95/C erst ganz zum Schluss zur Phase B zugegeben wird, um einen übermäßigen Lufteintrag zu vermeiden. Phase A bei 60 °C mit Hilfe der Dispergierscheibe langsam in Phase B (RT) einemulgieren. Phasen C und D zufügen, danach 4 min mit dem Ultra-Turrax T50, Stufe 4 homogenisieren. Auf Raumtemperatur abkühlen.
pH (23 °C) = 5,5 - 6,0
Viskosität: Brookfield DV II + Helipath, Spindel C, 5 Rpm, 24 °C=11200 mPa·s

### Bezugsquellen:

(1) Terry Laboratoires
(2) Alfa Aesar GmbH & Co. KG
(3) Merck KGaA/Rona®
(4) Zschimmer & Schwarz GmbH & Co.
(5) Nordmann, Rassmann GmbH & Co.
(6) Cognis GmbH
(7) Gustav Heess GmbH
(8) Evonik Goldschmidt GmbH
(9) Noveon
(10) Clariant GmbH
(11) BASF AG
(12) Parfex
(13) ISP Global Technologies

### Beispiel A6: Nagellack

| **Rohstoff** | | **INCI** | **[%]** |
|---|---|---|---|
| Pigmentgemisch aus Beispiel 8 | (1) | | 2,00 |
| Thixotrope Nagellack-Base 12897 | (2) | ETHYL ACETATE, BUTYL ACETATE, NITROCELLULOSE, PHTHALIC ANHYDRIDE/TRIMETLLITIC ANHYDRIDE/GLYCOLS COPOLYMER, ACETYL TRIBUTYL CITRATE, ISOPROPYL ALCOHOL, STEARALKON IUM HECTORITE, ADIPIC ACID/NEOPENTYL GLYCOL/TRIMELLITIC ANYHDRIDE COPLYMER | 98,00 |

### Herstellung:

Das Pigmentgemisch aus Beispiel 1 wird zusammen mit der Lackbase eingewogen, gut vermischt und anschließend 10 Minuten bei 1000 Upm gerührt.

### Bezugsquellen:

(1) Merck KGaA
(2) International Lacquers S.A.

### Beispiel A7: Lippenstift

| **Rohstoff** | | **INCI** | **[%]** |
|---|---|---|---|
| Phase A | | | |
| Pigmentgemisch aus Beispiel 4 | (1) | | 15,00 |

| Phase B | | | |
|---|---|---|---|
| Oxynex® K flüssig | (1) | PEG-8, TOCOPHEROL, ASCORBYL PALMITATE, ASCORBIC ACID, CITRIC ACID | 0,05 |
| Sensiva® PA 20 | (2) | PHENETHYL ALCOHOL, ETHYLHEXYL GLYCERIN | 1,00 |
| Paraffin viscous | (1) | PARAFFINUM LIQUIDUM | 2,10 |
| Adeps Lanae | (3) | LANOLIN | 3,50 |
| Paracera C 44 | (4) | COPERNICIA CERIFERA, CERESIN | 5,25 |
| Isopropyl Myristate | (5) | ISOPROPYL MYRISTATE | 5,60 |
| Wax white | (1) | CERA ALBA | 8,75 |
| Castor Oil | (3) | RICINUS COMMUNIS (CASTOR) SEED OIL | 58,75 |

### Herstellung:

Die Bestandteile der Phase B auf ca. 75 °C erhitzen bis alles geschmolzen ist. Phase A zugeben und gut durchrühren. Die Lippenstiftmasse auf 65 °C abkühlen und rühren bis die Phase frei von Luftblasen ist Die homogene Schmelze wird in die auf 55 °C vorgewärmten Gießformen gegossen. Anschließend kühlt man die Formen ab und entfernt die Gießlinge kalt. Nach Erwärmen der Lippenstifte auf Raumtemperatur werden die Lippenstifte kurz abgeflammt.

### Bezugsquellen:

(1) Merck KGaA
(2) Schülke & Mayr GmbH
(3) Henry Lamotte Oils GmbHSasol Germany GmbH
(4) Azelis Germany GmbH
(5) BASF AG

## Patentansprüche

1. Pigmentgemisch, **dadurch gekennzeichnet, dass** es mindestens zwei Komponenten A und B enthält, wobei
- Komponente A ein Gemisch aus plättchenförmigen und sphärischen Substraten ist, das mit ein oder mehreren anorganischen Schichten und/oder organischen Schichten belegt ist,
und
- Komponente B kristalline oder amorphe Partikel ausgewählt aus der Gruppe der Metalloxide, Metallhydroxide, Metalloxyhalogenide, Berliner Blau oder deren Gemische sind.

2. Pigmentgemisch nach Anspruch 1, **dadurch gekennzeichnet, dass** die plättchenförmigen Substrate ausgewählt sind aus der Gruppe natürlicher Glimmer, synthetischer Glimmer, Talkum, Kaolin, Glasplättchen, SiO₂-Plättchen, Al₂O₃-Plättchen, Graphit-Plättchen, Fe₂O₃-Plättchen, BiOCl, TiO₂-Plättchen, Nitrid-Plättchen, Oxinitrid-, BN-Plättchen, Fischsilber oder deren Gemische.

3. Pigmentgemisch nach Anspruch 1 oder 2, **dadurch gekennzeichnet, dass** die sphärischen Substrate ausgewählt sind aus der Gruppe Magnesiumsilikat, Aluminiumsilikat, Alkalialuminiumsilikat, Erdalkalialuminiumsilikat und deren Kombinationen, SiO₂-Kugeln, Glaskugeln, Glashohlkugeln, Aluminiumoxid, keramische Kugeln und polymere Kugeln aus Ethylen/Acrylsäure-Copolymeren, Ethylen/Methacrylat-Copolymeren, HDI/Trimethylol Hexyllactone-Copolymeren, Nylon, Polyacrylate, Polymethylmethacrylat-Copolymere, Polyethylen, Polymethylsilsesquioxane und deren Kombinationen.

4. Pigmentgemisch nach einem oder mehreren der Ansprüche 1 bis 3, **dadurch gekennzeichnet, dass** die Komponente B aus kristallinen oder amorphen Partikel von Fe₂O₃, TiO₂, Berliner Blau, Cr₂O₃, Fe₃O₄, FeOOH, BiOCI, Titansuboxiden und deren Gemische bestehen.

5. Pigmentgemisch nach einem oder mehreren der Ansprüche 1 bis 4, **dadurch gekennzeichnet, dass** die plättchenförmigen Substrate der Komponente A natürliche oder synthetische Glimmerplättchen sind.

6. Pigmentgemisch nach einem oder mehreren der Ansprüche 1 bis 5, **dadurch gekennzeichnet, dass** das sphärische Substrat der Komponente A SiO₂-Kugeln, Glaskugeln oder Glashohlkugeln sind.

7. Pigmentgemisch nach einem oder mehreren der Ansprüche 1 bis 6, **dadurch gekennzeichnet, dass** das Substratgemisch der Komponente A mit ein oder zwei Metalloxidschichten oder Gemischen aus Metalloxiden belegt ist.

8. Pigmentgemisch nach einem oder mehreren der Ansprüche 1 bis 7, **dadurch gekennzeichnet, dass** die anorganische Schicht der Komponente A ausgewählt ist aus der Gruppe TiO₂, Fe₂O₃, Fe₃O₄, FeOOH, Cr₂O₃, Berliner Blau, Karminrot, Titansuboxid, SnO₂, ZnO, Al₂O₃, SiO₂ oder Gemische davon.

9. Pigmentgemisch nach einem oder mehreren der Ansprüche 1 bis 8, **dadurch gekennzeichnet, dass** die Komponente A folgende Zusammensetzung auf der Oberfläche des Substratgemisches aufweist:
Substratgemisch + Fe₂O₃ (1. Schicht) + SnO₂ (2. Schicht)
Substratgemisch + Fe₂O₃ (1. Schicht) + SiO₂ (2. Schicht)
Substratgemisch + Fe₃O₄ (1. Schicht) + TiO₂ (2. Schicht)
Substratgemisch + Fe₃O₄ (1. Schicht) + SnO₂ (2. Schicht)
Substratgemisch + Fe₃O₄ (1. Schicht) + SiO₂ (2. Schicht)
Substratgemisch + Cr₂O₃ (1. Schicht) + TiO₂ (2. Schicht)
Substratgemisch + Cr₂O₃ (1. Schicht) + SnO₂ (2. Schicht)
Substratgemisch + Cr₂O₃ (1. Schicht) + SiO₂ (2. Schicht)
Substratgemisch + TiO₂ (1. Schicht) + Berliner Blau (2. Schicht)
Substratgemisch + TiO₂ (1. Schicht) + Karminrot (2. Schicht)
Substratgemisch + SnO₂ (1. Schicht) + TiO₂ (2. Schicht)
Substratgemisch + TiO₂ (1. Schicht)
Substratgemisch + Fe₂O₃ (1. Schicht)
Substratgemisch + FeOOH (1. Schicht)
Substratgemisch + TiO₂ (1. Schicht) + Fe₂O₃ (2. Schicht)
Substratgemisch + Berliner Blau (1. Schicht)
Substratgemisch + Cr₂O₃ (1. Schicht)
Substratgemisch + Fe₂O₃ (1. Schicht) + TiO₂ (2. Schicht) + SiO₂ (3. Schicht)
Substratgemisch + TiO₂ (1. Schicht) + SiO₂ (2. Schicht) + TiO₂ (3. Schicht).

10. Pigmentgemisch nach einem oder mehreren der Ansprüche 1 bis 9, **dadurch gekennzeichnet, dass** die Komponenten A und B im Massenverhältnis (Gewichtsteile) 99 : 1 bis 1 : 99 gemischt werden.

11. Pigmentgemisch nach einem oder mehreren der Ansprüche 1 bis 10, **dadurch gekennzeichnet, dass** die Komponente A und/oder die Komponente B zur Erhöhung der Licht-, Temperatur- und Wetterstabilität zusätzlich eine äußere Schutzschicht aufweist.

12. Verfahren zur Herstellung des Pigmentgemisches nach einem oder mehreren der Ansprüche 1 bis 11, **dadurch gekennzeichnet, dass** die plättchenförmigen und sphärischen Substrate der Komponente A jeweils durch nasschemische Beschichtung oder im CVD- oder PVD-Verfahren mit einer oder mehreren anorganischen Schichten belegt werden und nachfolgend die Komponente A und die Komponente B miteinander gemischt und anschließend getrocknet werden und gegebenenfalls gemeinsam geglüht werden.

13. Verfahren nach Anspruch 12, **dadurch gekennzeichnet, dass** die plättchenförmigen und sphärischen Substrate der Komponente A gemischt werden und nasschemisch mit ein oder mehreren absorbierenden oder nicht absorbierenden Schichten belegt werden und das beschichtete Substrat/Kugel-Gemisch (Komponente A) mit den kristallinen oder amorphen Partikeln (Komponente B) gemischt, und das Gemisch aus den Komponenten A und B gemeinsam aufarbeitet wird, und das finale Gemisch bei Temperaturen von 150 - 1000 °C geglüht wird, wobei die Glühung gegebenenfalls unter Reduziergas erfolgt.

14. Verfahren nach Anspruch 12 oder 13, **dadurch gekennzeichnet, dass** bei der nasschemischen Beschichtung zu der wässrigen Suspension der Komponente A die wässrige Suspension der Komponente B gegeben wird und die Suspension enthaltend Komponenten A und B aufgearbeitet wird, oder dass nach der nasschemischen Belegung des Substratgemisches der Komponente A das Produkt abgetrennt und aufgearbeitet wird und anschließend das getrocknete Produkt der Komponente A mit der Suspension der Komponente B gemischt wird und die Suspension dann aufgearbeitet wird, oder dass zu der Suspension der Komponente B das Substratgemisch der Komponente A gegeben wird und das Gemisch aus Komponente B und Substratgemisch der Komponente A gemeinsam nasschemisch mit ein oder mehreren anorganischen Schichten belegt wird, und dann aufgearbeitet wird.

15. Verwendung des Pigmentgemisches nach einem oder mehreren der Ansprüche 1 bis 11 in Farben, Lacken, Druckfarben, Sicherheitsdruckfarben, Kunststoffen, keramischen Materialien, Glasuren, Gläsern, in kosmetischen Formulierungen, als Tracer, als Füllstoff und zur Herstellung von Pigmentpräparationen und Trockenpräparaten.

16. Formulierungen enthaltend ein Pigmentgemisch nach einem oder mehreren der Ansprüche 1 bis 11.

17. Formulierungen nach Anspruch 16, **dadurch gekennzeichnet, dass** sie neben dem Pigmentgemisch mindestens einen Bestandteil ausgewählt aus der Gruppe der Absorptionsmittel, Adstringenzien, antimikrobiellen Stoffe, Antioxidantien, Antiperspirantien, Antischaummitteln, Antischuppenwirkstoffe, Antistatika, Bindemittel, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Füllstoffen, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Aloe Vera, Avocadoöl, Coenzym Q10, grüner Tee Extrakt, Viskositätsreglern, Parfüm und Vitaminen enthalten.

## Claims

1. Pigment mixture, **characterised in that** it comprises at least two components A and B, where
- component A is a mixture of flake-form and spherical substrates which is covered with one or more inorganic layers and/or organic layers,
and
- component B comprises crystalline or amorphous particles selected from the group of the metal oxides, metal hydroxides, metal oxyhalides, Berlin Blue or mixtures thereof.

2. Pigment mixture according to Claim 1, **characterised in that** the flake-form substrates are selected from the group natural mica, synthetic mica, talc, kaolin, glass flakes, SiO₂ flakes, Al₂O₃ flakes, graphite flakes, Fe₂O₃ flakes, BiOCI, TiO₂ flakes, nitride flakes, oxynitride flakes, BN flakes, pearl essence or mixtures thereof.

3. Pigment mixture according to Claim 1 or 2, **characterised in that** the spherical substrates are selected from the group magnesium silicate, aluminium silicate, alkali metal aluminium silicate, alkaline-earth metal aluminium silicate and combinations thereof, SiO₂ spheres, glass spheres, hollow glass spheres, aluminium oxide, ceramic spheres and polymeric spheres comprising ethylene-acrylic acid copolymers, ethylene-methacrylate copolymers, HDI-trimethylolhexyl lactone copolymers, nylon, polyacrylates, polymethyl methacrylate copolymers, polyethylene, polymethylsilsesquioxanes and combinations thereof.

4. Pigment mixture according to one or more of Claims 1 to 3, **characterised in that** component B consists of crystalline or amorphous particles of Fe₂O₃, TiO₂, Berlin Blue, Cr₂O₃, Fe₃O₄, FeOOH, BiOCI, titanium suboxides and mixtures thereof.

5. Pigment mixture according to one or more of Claims 1 to 4, **characterised in that** the flake-form substrates of component A are natural or synthetic mica flakes.

6. Pigment mixture according to one or more of Claims 1 to 5, **characterised in that** the spherical substrate of component A comprises SiO₂ spheres, glass spheres or hollow glass spheres.

7. Pigment mixture according to one or more of Claims 1 to 6, **characterised in that** the substrate mixture of component A is covered with one or two metal oxide layers or mixtures of metal oxides.

8. Pigment mixture according to one or more of Claims 1 to 7, **characterised in that** the inorganic layer of component A is selected from the group TiO₂, Fe₂O₃, Fe₃O₄, FeOOH, Cr₂O₃, Berlin Blue, Carmine Red, titanium suboxide, SnO₂, ZnO, Al₂O₃, SiO₂ or mixtures thereof.

9. Pigment mixture according to one or more of Claims 1 to 8, **characterised in that** component A has the following composition on the surface of the substrate mixture:
substrate mixture + Fe₂O₃ (1st layer) + SnO₂ (2nd layer)
substrate mixture + Fe₂O₃ (1st layer) + SiO₂ (2nd layer)
substrate mixture + Fe₃O₄ (1st layer) + TiO₂ (2nd layer)
substrate mixture + Fe₃O₄ (1st layer) + SnO₂ (2nd layer)
substrate mixture + Fe₃O₄ (1st layer) + SiO₂ (2nd layer)
substrate mixture + Cr₂O₃ (1st layer) + TiO₂ (2nd layer)
substrate mixture + Cr₂O₃ (1st layer) + SnO₂ (2nd layer)
substrate mixture + Cr₂O₃ (1st layer) + SiO₂ (2nd layer)
substrate mixture + TiO₂ (1st layer) + Berlin Blue (2nd layer)
substrate mixture + TiO₂ (1st layer) + Carmine Red (2nd layer)
substrate mixture + SnO₂ (1st layer) + TiO₂ (2nd layer)
substrate mixture + TiO₂ (1st layer)
substrate mixture + Fe₂O₃ (1st layer)
substrate mixture + FeOOH (1st layer)
substrate mixture + TiO₂ (1st layer) + Fe₂O₃ (2nd layer)
substrate mixture + Berlin Blue (1st layer)
substrate mixture + Cr₂O₃ (1st layer)
substrate mixture + Fe₂O₃ (1st layer) + TiO₂ (2nd layer) + SiO₂ (3rd layer)
substrate mixture + TiO₂ (1st layer) + SiO₂ (2nd layer) + TiO₂ (3rd layer).

10. Pigment mixture according to at least one of Claims 1 to 9, **characterised in that** components A and B are mixed in the weight ratio (parts by weight) 99 : 1 to 1 : 99.

11. Pigment mixture according to one or more of Claims 1 to 10, **characterised in that** component A and/or component B additionally has an outer protective layer in order to increase the light, temperature and weather stability.

12. Process for the preparation of the pigment mixture according to one or more of Claims 1 to 11, **characterised in that** the flake-form and spherical substrates of component A are in each case covered with one or more inorganic layers by wet-chemical coating or by the CVD or PVD process, and component A and component B are subsequently mixed with one another and subsequently dried and optionally jointly calcined.

13. Process according to Claim 12, **characterised in that** the flake-form and spherical substrates of component A are mixed and covered with one or more absorbent or non-absorbent layers by wet-chemical methods, and the coated substrate/sphere mixture (component A) is mixed with the crystalline or amorphous particles (component B), and the mixture of components A and B is worked up jointly, and the final mixture is calcined at temperatures of 150 - 1000°C, where the calcination is optionally carried out under reducing gas.

14. Process according to Claim 12 or 13, **characterised in that**, in the case of wet-chemical coating, the aqueous suspension of component B is added to the aqueous suspension of component A and the suspension comprising components A and B is worked up, or **in that**, after the wet-chemical covering of the substrate mixture of component A, the product is separated off and worked up and the dried product of component A is subsequently mixed with the suspension of component B and the suspension is then worked up, or **in that** the substrate mixture of component A is added to the suspension of component B and the mixture of component B and substrate mixture of component A is jointly covered with one or more inorganic layers by wet-chemical methods and then worked up.

15. Use of the pigment mixture according to one or more of Claims 1 to 11 in paints, coatings, printing inks, security printing inks, plastics, ceramic materials, glazes, glasses, in cosmetic formulations, as tracer, as filler and for the preparation of pigment preparations and dry preparations.

16. Formulations comprising a pigment mixture according to one or more of Claims 1 to 11.

17. Formulations according to Claim 16, **characterised in that**, besides the pigment mixture, they comprise at least one constituent selected from the group of the absorbents, astringents, antimicrobial substances, antioxidants, antiperspirants, antifoaming agents, antidandruff active compounds, antistatics, binders, biological additives, bleaches, chelating agents, deodorisers, emollients, emulsifiers, emulsion stabilisers, dyes, humectants, film formers, fillers, fragrances, flavours, insect repellents, preservatives, anticorrosion agents, cosmetic oils, solvents, oxidants, vegetable constituents, buffer substances, reducing agents, surfactants, propellant gases, opacifiers, UV filters and UV absorbers, denaturing agents, aloe vera, avocado oil, coenzyme Q10, green tea extract, viscosity regulators, perfume and vitamins.

## Revendications

1. Mélange de pigments, **caractérisé en ce qu'**il comprend au moins deux composants A et B, où :
- le composant A est un mélange de substrats sous forme de flocons et sphériques qui est recouvert d'une ou de plusieurs couche(s) inorganique(s) et/ou d'une ou de plusieurs couche(s) organique(s) ;
et
- le composant B comprend des particules cristallines ou amorphes qui sont sélectionnées parmi le groupe qui est constitué par les oxydes de métaux, les hydroxydes de métaux, les oxyhalogénures de métaux, le bleu de Berlin ou des mélanges afférents.

2. Mélange de pigments selon la revendication 1, **caractérisé en ce que** les substrats sous forme de flocons sont sélectionnés parmi le groupe qui est constitué par le mica naturel, le mica synthétique, le talc, le kaolin, les flocons de verre, les flocons de SiO₂, les flocons d'Al₂O₃, les flocons de graphite, les flocons de Fe₂O₃, le BiOCl, les flocons de TiO₂, les flocons de nitrure, les flocons d'oxynitrure, les flocons de BN, l'essence de perle ou des mélanges afférents.

3. Mélange de pigments selon la revendication 1 ou 2, **caractérisé en ce que** les substrats sphériques sont sélectionnés parmi le groupe qui est constitué par le silicate de magnésium, le silicate d'aluminium, le silicate d'aluminium et de métaux alcalins, le silicate d'aluminium et de métaux alcalino-terreux et des combinaisons afférentes, les sphères de SiO₂, les sphères de verre, les sphères de verre creuses, l'oxyde d'aluminium, les sphères de céramique et les sphères polymériques comprenant des copolymères éthylène-acide acrylique, des copolymères éthylène-méthacrylate, des copolymères HDI-lactone de triméthylolhexyle, le nylon, les polyacrylates, les copolymères de polyméthylméthacrylate, le polyéthylène, les polyméthylsilsesquioxanes et des combinaisons afférentes.

4. Mélange de pigments selon une ou plusieurs des revendications 1 à 3, **caractérisé en ce que** le composant B est constitué par des particules cristallines ou amorphes de Fe₂O₃, de TiO₂, de bleu de Berlin, de Cr₂O₃, de Fe₃O₄, de FeOOH, de BiOCl, de sous-oxydes de titane et par des mélanges afférents.

5. Mélange de pigments selon une ou plusieurs des revendications 1 à 4, **caractérisé en ce que** les substrats sous forme de flocons du composant A sont des flocons de mica naturel ou synthétique.

6. Mélange de pigments selon une ou plusieurs des revendications 1 à 5, **caractérisé en ce que** le substrat sphérique du composant A comprend des sphères de SiO₂, des sphères de verre ou des sphères de verre creuses.

7. Mélange de pigments selon une ou plusieurs des revendications 1 à 6, **caractérisé en ce que** le mélange de substrats du composant A est recouvert par une ou deux couche(s) d'oxyde(s) métallique(s) ou par des mélanges d'oxydes métalliques.

8. Mélange de pigments selon une ou plusieurs des revendications 1 à 7, **caractérisé en ce que** la couche inorganique du composant A est sélectionnée parmi le groupe qui est constitué par le TiO₂, le Fe₂O₃, le Fe₃O₄, le FeOOH, le Cr₂O₃, le bleu de Berlin, le rouge carmin, un sous-oxyde de titane, le SnO₂, le ZnO, l'Al₂O₃, le SiO₂ ou des mélanges afférents

9. Mélange de pigments selon une ou plusieurs des revendications 1 à 8, **caractérisé en ce que** le composant A présente la composition qui suit sur la surface du mélange de substrats :
mélange de substrats + Fe₂O₃ (1ère couche) + SnO₂ (2ème couche)
mélange de substrats + Fe₂O₃ (1ère couche) + SiO₂ (2ème couche)
mélange de substrats + Fe₃O₄ (1ère couche) + TiO₂ (2ème couche)
mélange de substrats + Fe₃O₄ (1ère couche) + SnO₂ (2ème couche)
mélange de substrats + Fe₃O₄ (1ère couche) + SiO₂ (2ème couche)
mélange de substrats + Cr₂O₃ (1ère couche) + TiO₂ (2ème couche)
mélange de substrats + Cr₂O₃ (1ère couche) + SnO₂ (2ème couche)
mélange de substrats + Cr₂O₃ (1ère couche) + SiO₂ (2ème couche)
mélange de substrats + TiO₂ (1ère couche) + bleu de Berlin (2ème couche)
mélange de substrats + TiO₂ (1ère couche) + rouge carmin (2ème couche)
mélange de substrats + SnO₂ (1ère couche) + TiO₂ (2ème couche)
mélange de substrats + TiO₂ (1ère couche)
mélange de substrats + Fe₂O₃ (1ère couche)
mélange de substrats + FeOOH (1ère couche)
mélange de substrats + TiO₂ (1ère couche) + Fe₂O₃ (2ème couche)
mélange de substrats + bleu de Berlin (1ère couche)
mélange de substrats + Cr₂O₃ (1ère couche)
mélange de substrats + Fe₂O₃ (1ère couche) + TiO₂ (2ème couche) + SiO₂ (3^{ème} couche)
mélange de substrats + TiO₂ (1ère couche) + SiO₂ (2ème couche) + TiO₂ (3^{ème} couche).

10. Mélange de pigments selon au moins l'une des revendications 1 à 9, **caractérisé en ce que** les composants A et B sont mélangés selon le rapport en poids (parties en poids) de 99 : 1 à 1 : 99.

11. Mélange de pigments selon une ou plusieurs des revendications 1 à 10, **caractérisé en ce que** le composant A et/ou le composant B comporte(nt) de façon additionnelle une couche de protection externe afin d'augmenter la stabilité à la lumière, à la température et aux conditions météorologiques.

12. Procédé pour la préparation du mélange de pigments selon une ou plusieurs des revendications 1 à 11, **caractérisé en ce que** les substrats sous forme de flocons et sphériques du composant A sont dans chaque cas recouverts d'une ou de plusieurs couche(s) inorganique(s) un moyen d'un revêtement chimique par voie humide ou au moyen du procédé CVD ou PVD, et le composant A et le composant B sont ensuite mélangés l'un avec l'autre et sont ensuite séchés et en option calcinés de façon conjointe.

13. Procédé selon la revendication 12, **caractérisé en ce que** les substrats sous forme de flocons et sphériques du composant A sont mélangés et recouverts d'une ou de plusieurs couche(s) absorbante(s) ou non absorbante(s) au moyen de procédés chimiques par voie humide, et le mélange de substrats/sphères revêtu (le composant A) est mélangé avec les particules cristallines ou amorphes (le composant B), et le mélange de composants A et B fait l'objet d'un traitement conclusif/est élaboré de façon conjointe, et le mélange final est calciné à des températures de 150 - 1000°C, dans lequel la calcination est en option mise en oeuvre sous un gaz réducteur.

14. Procédé selon la revendication 12 ou 13, **caractérisé en ce que**, dans le cas d'un revêtement chimique par voie humide, la suspension aqueuse du composant B est ajoutée à la suspension aqueuse du composant A et la suspension qui comprend les composants A et B est élaborée, ou **en ce que**, après le recouvrement chimique par voie humide du mélange de substrats du composant A, le produit est séparé et est élaboré et le produit séché du composant A est ensuite mélangé avec la suspension du composant B et la suspension est ensuite élaborée, ou **en ce que** le mélange de substrats du composant A est ajouté à la suspension du composant B et le mélange du composant B et du mélange de substrats du composant A est ensuite recouvert de façon conjointe d'une ou de plusieurs couche(s) inorganique(s) au moyen de procédés chimiques par voie humide puis est élaboré.

15. Utilisation du mélange de pigments selon une ou plusieurs des revendications 1 à 11 dans les peintures, les revêtements, les encres d'impression, les encres d'impression de sécurité, les matières plastiques, les matériaux de céramique, les vernis, les verres, dans les formulations cosmétiques, telles que les moyens de contourage, telles que les moyens de comblement et pour la préparation de préparations de pigments et de préparations sèches.

16. Formulations comprenant un mélange de pigments selon une ou plusieurs des revendications 1 à 11.

17. Formulations selon la revendication 16, **caractérisées en ce que**, en plus du mélange de pigments, elles comprennent au moins un constituant qui est sélectionné parmi le groupe qui est constitué par les agents d'absorption, les astringents, les substances antimicrobiennes, les antioxydants, les anti-transpirant, les agents anti-moussage, les composés actifs antipelliculaires, les antistatiques, les agents de liaison, les additifs biologiques, les agents blanchissants, les agents chélatants, les désodorisants, les émollients, les émulsifiants, les stabiliseurs d'émulsion, les colorants, les agents d'humidification, les agents de formation de film, les agents de comblement, les fragrances, les arômes, les répulsifs pour insectes, les conservateurs, les agents anticorrosion, les huiles cosmétiques, les solvants, les oxydants, les composants végétaux, les substances tampon, les agents de réduction, les agents tensioactifs, les gaz propulseurs, les opacifiants, les filtres UV et les absorbeurs d'UV, les agents dénaturants, l'aloe vera, l'huile d'avocat, le coenzyme Q10, l'extrait de thé vert, les régulateurs de viscosité, les parfums et les vitamines.
